# EUROPÄISCHE PATENTSCHRIFT

(11) **EP 0 888 356 B1**
(45) Veröffentlichungstag und Bekanntmachung des Hinweises auf die Patenterteilung: **20.03.2002**
(21) Anmeldenummer: 97916315.1
(22) Anmeldetag: 29.01.1997
(51) Int. Cl.: C07D 491/14

(54) **KONDENSIERTE 2,3-BENZODIAZEPIN DERIVATE UND DEREN VERWENDUNG ALS AMPA-REZEPTOREN-HEMMER**
CONDENSED 2,3-BENZODIAZEPINE DERIVATIVES AND THEIR USE AS AMPA-RECEPTOR INHIBITORS
DERIVES DE 2,3-BENZODIAZEPINE CONDENSES ET LEUR UTILISATION COMME INHIBITEURS DE RECEPTEURS D'AMPA

(30) Priorität: 01.02.1996 DE 19604919
(43) Veröffentlichungstag der Anmeldung: 07.01.1999
(73) Patentinhaber: SCHERING AKTIENGESELLSCHAFT, 13353 Berlin (DE)
(72) Erfinder: CSUZDI, Ernese, H-1046 Budapest (HU); HAMORI, Tamás, H-1031 Budapest (HU); ABRAHAM, Gizella, H-1118 Budapest (HU); SOLYOM, Sándor, H-1144 Budapest (HU); TARNAWA, István, H-1147 Budapest (HU); BERZSENYI, Pál, H-1174 Budapest (HU); ANDRASI, Ferenc, H-1125 Budapest (HU); LING, István, H-1141 Budapest (HU); SIMAY, Antal, H-1124 Budapest (HU); GAL, Melinda, H-1142 Budapest (HU); HORVATH, Katalin, H-1025 Budapest (HU); SZENTKUTI, Eszter, H-1165 Budapest (HU); SZÖLLOSY, Márta, H-1105 Budapest (HU); PALLAGI, István, H-1157 Budapest (HU)
(86) Internationale Anmeldenummer: DE9700234
(87) Internationale Veröffentlichungsnummer: WO9728163

(56) Entgegenhaltungen:
- EUROPEAN JOURNAL OF PHARMACOLOGY, Bd. 294, 1995, AMSTERDAM NL, Seiten 411-22, XP002034232 G. DE SARRO ET AL.: "GYKI 52466 and related 2,3-benzodiazepines as anticonvulsant agents in DBA/2 mice"

## Beschreibung

Die Erfindung betrifft neue 2,3-Benzodiazepinderivate, deren Herstellung und Verwendung als Arzneimittel.

Es ist bereits aus Eur. J. Pharmacol., 294 (1995), 411-22 bekannt, daß ausgewählte 2,3-Benzodiazepinderivate modulatorische Aktivität an Quisqualat-Rezeptoren besitzen und sich aufgrund dieser Eigenschaft als Arzneimittel zur Behandlung von Krankheiten des zentralen Nervensystems eignen.

Es wurde nun gefunden, daß die erfindungsgemäßen 2,3-Benzodiazepinderivate ebenfalls zur Behandlung von Krankheiten des zentralen Nervensystems geeignet sind, wobei die Verbindungen sich gegenüber dem genannten Stand der Technik durch bessere Eigenschaften auszeichnen.

Die Erfindung betrifft die Verbindungen der Formel I 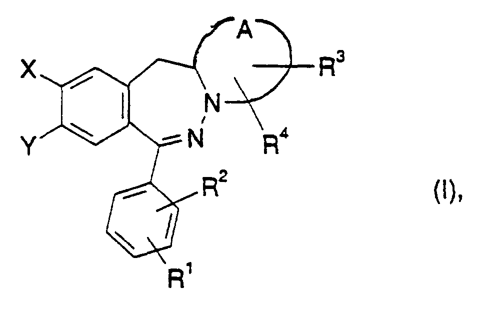 worin
R¹ und R² gleich oder verschieden sind und Wasserstoff, C₁ - C₆-Alkyl, Nitro, Halogen, Cyano, die Gruppe -NR⁸R⁹, -O-C₁₋₄-Alkyl, -CF₃, OH oder C₁₋₆-Alkanoyloxy,
R³ und R⁴gleich oder verschieden sind und Wasserstoff, Halogen, C₁-C₆-Alkoxy, Hydroxy,Thiocyanato, C₁-C₆-Alkylthio, Cyano, COOR¹², PO₃R¹³R¹⁴, C₁₋₆-Alkanoyl, C₁₋₆-Alkanoyloxy, gegebenenfalls mit C₁₋₄-Alkoxy oder Phenyl substituiertes C₂₋₆-Alkynyl, gegebenenfalls mit C₁₋₄-Alkoxy oder Phenyl substituiertes C₂₋₆-Alkenyl, gegebenenfalls durch Halogen, Hydroxy, C₁-C₆-Alkoxy, C₁-C₆-Thioalkyl, NR¹⁰R¹¹ substituiertes C₁ - C₆-Alkyl, C₃₋₇-Cycloalkyl oder einen gegebenenfalls mit Halogen, C₁₋₄-Alkyl und C₁₋₄-Alkoxy substituiertenAryl- oder Hetaryl-Rest,
R⁸ und R⁹ gleich oder verschieden sind und Wasserstoff, C₁ - C₆-Alkyl oder die Gruppe -CO-C₁₋₆-Alkyl,
R¹⁰ und R¹¹ gleich oder verschieden sind und Wasserstoff, C₁ - C₆-Alkyl oder C₁₋₆-Alkanoyl oder gemeinsam mit dem Stickstoffatom einen 5-7-gliedrigen gesättigten Heterocyclus bilden, der ein weiteres Sauerstoff-, Schwefel- oder Stickstoffatom enthalten und mit C₁₋₄-Alkyl und Phenyl substituiert sein kann,
R¹², R,¹³, R¹⁴ gleich oder verschieden sind und H oder C₁-C₆-Alkyl,
X Wasserstoff oder Halogen,
Y C₁₋₆-Alkoxy oder X und Y gemeinsam -O-(CH₂)ₙ-O-,
n 1, 2 oder 3 bedeuten und

A gemeinsam mit dem Stickstoff einen gesättigten oder ungesättigten fünfgliedrigen Heterocyclus bildet, der 1-3 Stickstoffatome und/oder ein Sauerstoffatom und/oder eine oder zwei Carbonylgruppen enthalten kann oder deren Isomere oder physiologisch verträglichen Salze.

Unter Alkyl ist jeweils ein geradkettiger oder verzweigter Alkylrest wie beispielsweise Methyl, Ethyl, Propyl, Isopropyl, Butyl, Isobutyl, sek. Butyl, Pentyl, Isopentyl oder Hexyl zu verstehen.

R³ und R⁴ in der Bedeutung C₂₋₆-Alkenyl enthalten mindestens eine Doppelbindung wie beispielsweise Vinyl, Propenyl, Buten-1-yl, Isobutenyl, Penten-1-yl, 2,2-Dimethyl-buten-1-yl, 3-Methylbuten-1-yl, Hexen-1-yl. Bedeuten R³ oder R⁴ C₂₋₆-Alkynyl, so ist mindestens 1 Dreifachbindung vorhanden, wie beispielsweise Ethynyl, Propynyl, Butyn-1-yl, Butyn-2-yl, Pentyn-1-yl, Pentyn-2-yl, 3-Methylbutyn-1-yl, Hexyn-1-yl. Der Alkenyl-und Alkinylrest kann substituiert sein, z.B. mit C₁₋₄-Alkoxy oder Phenyl, das substituiert sein kann mit Halogen. Liegt ein halogenierter Alkylrest vor, so kann dieser ein- oder mehrfach halogeniert bzw. perhalogeniert sein wie CF₃.

Unter Halogen ist jeweils Fluor, Chlor, Brom und Jod zu verstehen.

Der Aryl- und Hetarylrest R³ und R⁴ kann ein- bis dreifach gleich oder verschieden mit Halogen, C₁₋₄-Alkoxy oder C₁₋₄-Alkyl substituiert sein.

Der Aryl- und Hetarylrest kann als Mono- oder Bicyclus vorliegen und 5-12 Ringatome, vorzugsweise 5-9 Ringatome enthalten wie beispielsweise Phenyl, Biphenyl, Naphthyl, Indenyl als Arylrest und Thienyl, Furyl, Pyranyl, Pyrrolyl, Pyrazolyl, Pyridyl, Pyrimidinyl, Pyridazinyl, Oxazolyl, Iso-oxazolyl, Thiazolyl, Isothiazolyl, 1,3,4-Oxadiazol-2-yl, 1,2,4-Oxadiazolyl-5-yl, 1,2,4-Oxadiazol-3-yl, Chinolyl, Isochinolyl, Benzo[1]thienyl, Benzofuranyl als Hetarylrest mit 1-3 Heteroatomen wie Schwefel, Sauerstoff und/oder Stickstoff. Als bevorzugt seien 2-Thienyl, 3-Thienyl, Pyridin-2-yl, Pyridin-3-yl, Pyridin-4-yl und Phenyl genannt.

Unter Cycloalkyl ist jeweils Cyclopropyl, Cyclobutyl, Cyclopentyl, Cyclohexyl und Cycloheptyl gemeint, insbesondere C₃₋₅-Cycloalkyl.

Als Alkanoylreste sind jeweils geradkettige oder verzweigte aliphatische Carbonsäurereste geeignet wie Formyl, Acetyl, Propionyl, Butanoyl, Isopropylcarbonyl, Caproyl, Valeroyl, Trimethylacetyl u.a..

Bilden R¹⁰ und R¹¹ gemeinsam mit dem Stickstoffatom einen Heterocyclus, so ist beispielsweise genannt Piperidin, Pyrrolidin, Thiomorpholin, Hexahydroazepin, Morpholin, Piperazin, Imidazolidin, Hexahydrodiazepin. Ist der Heterocyclus substituiert, so kann der Substituent C₁₋₄-Alkyl oder Phenyl 1-2 fach stehen wie beispielsweise N-Methyl-piperazin, N-Phenyl-piperazin, 2,6-Dimethylmorpholin.

Bildet A gemeinsam mit dem Stickstoffatom einen gesättigten Heterocyclus, so kann dieser an den Kohlenstoffatomen oder an einem weiteren Stickstoffatom substituiert sein. In diesem Fall bedeutet A beispielsweise C₃-Alkylen, das mit R³ und R⁴ substituiert sein kann und bei dem 1,2 oder 3 Alkylengruppen durch Sauerstoff, Carbonyl oder -NR³-ersetzt sein können wie beispielsweise -(CH₂)₃-, -CH₂-NR³-CH₂_, -CH₂-O-CH₂-, -CH₂-O-CO-, -CH₂-NR³-CO-, -CO-NR³-CO- oder CH₂-O-CR³R⁴, wobei die Carbonylgruppe an das Stickstoffatom des Benzodiazepins gebunden ist und R³ und R⁴ vorzugsweise C₁₋ ₄-Alkyl bedeuten. Diese Verbindungen der Formel I enthalten ein chirales Zentrum in 4-Stellung des 2,3-Benzodiazepingerüstes und können als Racemat oder optische Isomere vorliegen.

Bildet A gemeinsam mit dem Stickstoffatom einen ungesättigten 5-gliedrigen Heterocyclus, so ist in Position 4 des 2,3-Benzodiazepingerüstes kein chirales Kohlenstoffatom vorhanden sondern eine exocyclische Doppelbindung. Der ungesättigte 5-gliedrige Heterocyclus kann teilweise ungesättigt oder aromatisch vorliegen. Bevorzugt sind Heteroaromaten mit 1-3 Stickstoffatomen, worin A beispielsweise die folgende Bedeutung hat:

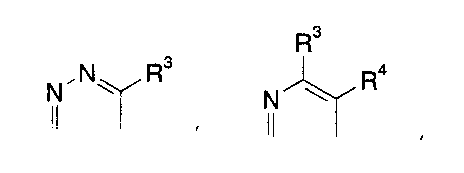

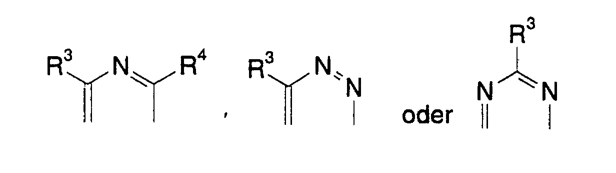

Die physiologisch verträglichen Salze leiten sich von anorganischen und organischen Säuren ab. Geeignet sind anorganische Säuren wie beispielsweise Halogenwassertoffsäuren wie Chlorwasserstoffsäure, Bromwasserstoffsäure, Phosphorsäure, Schwefelsäure oder organische Säuren wie beispielsweise aliphatische oder aromatische Mono- oder Dicarbonsäuren wie Ameisensäure, Essigsäure, Maleinsäure, Fumarsäure, Succinsäure, Milchsäure, Weinsäure, Zitronensäure, Oxalsäure, Glyoxylsäure oder Sulfonsäuren, beispielsweise C₁₋₄-Alkansulfonsäuren wie Methansulfonsäure oder gegebenenfalls durch Halogen oder C₁₋₄-Alkyl substituierte Benzolsulfonsäuren wie p-Toluolsulfonsäure.

Die Verbindungen der Formel I umfassen auch alle möglichen Stereoisomeren und deren Gemische wie Diastereomeren, Racemate und Enantiomeren.

Bevorzugt sind Verbindungen der allgemeinen Formel I worin R² Wasserstoff bedeutet.

Die Verbindungen der allgemeinen Formel I sowie deren physiologisch verträglichen Salze sind aufgrund ihrer nichtkompetitiven Hemmung der AMPA-Rezeptoren als Arzneimittel verwendbar. Aufgrund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als nichtkompetitive Antagonisten excitatorischer Aminosäuren wirken, eignen sie sich insbesondere zur Behandlung von solchen Krankheiten, die über die Rezeptoren excitatorischer Aminosäuren, insbesondere den AMPA-Rezeptor, beeinflußt werden .

Die pharmakologische Wirksamkeit der Verbindungen der Formel I wurde mittels der nachfolgend beschriebenen Teste bestimmt:

Männliche NMRI Mäuse mit einem Gewicht von 18-22 g wurden unter kontrollierten Verhältnissen (6°°-18°° Uhr Hell/Dunkelrythmus, bei freiem Zugang zu Futter und Wasser) gehalten und ihre Zuordnung zu Gruppen wurde randomisiert. Die Gruppen bestanden aus 5 - 16 Tieren. Die Beobachtung der Tiere wurde zwischen 8°° und 13°° Uhr vorgenommen.

AMPA wurde in den linken Ventrikel von frei beweglichen Mäusen gespritzt. Der Applikator bestand aus einer Kanüle mit einer Vorrichtung aus rostfreiem Stahl, die die Tiefe der Injektion auf 3,2 mm begrenzt. Der Applikator war an eine Injektionspumpe angeschlossen. Die Injektionsnadel wurde perpendicular zu der Oberfläche des Schädels nach den Koordinaten von Montemurro und Dukelow eingeführt. Die Tiere wurden bis zum Auftreten von clonischen bzw. tonischen Krämpfen bis zu 180 sec. beobachtet. Die clonischen Bewegungen, die länger als 5 sec. andauern, wurden als Krämpfe gezählt. Der Anfang der clonischen Krämpfe wurde als Endpunkt für die Bestimmung der Krampfschwelle verwendet. Die Dosis, die notwendig war, um die Krampfschwelle um 50% herauf- bzw. herabzusetzen (THRD₅₀) wurde in 4-5 Experimenten bestimmt. Die THRD₅₀ und die Vertrauensgrenze wurde in einer Regressionsanalyse bestimmt.

Die Ergebnisse dieser Versuche zeigen, daß die Verbindung der Formel I und deren Säureadditionssalze funktionelle Störungen des AMPA-Rezeptors beeinflussen. Sie eignen sich daher zu Herstellung von Arzneimitteln zur symptomatischen und präventiven Behandlung von Erkrankungen, die durch Veränderung der Funktion des AMPA-Rezeptor-Komplexes ausgelöst werden.
Die Behandlung mit den erfindungsgemäßen Verbindungen verhindert bzw. verzögert die infolge der Erkrankung auftretenden Zellschädigungen und funktionellen Störungen und vermindert die dadurch entstehenden Symptome.

Erfindungsgemäß können die Verbindungen verwendet werden zur Behandlung neurologischer und psychiatrischer Störungen, die durch die Überstimulation des AMPA-Rezeptors ausgelöst werden. Zu den neurologischen Erkrankungen , die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäss können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Aids Demenz, neurologischer Symptome, die mit HIV-Infektionen zusammenhängen, Multiinfarkt Demenz sowie Epilepsie und Muskelspastik verwendet werden. Zu den psychiatrischen Erkrankungen gehören Angstzustände, Schizophrenie, Migräne, Schmerzzustände, sowie die Behandlung von Schlafstörungen und der Entzugssymptomatik nach Drogenmißbrauch wie bei Alkohol-, Kokain-, Benzodiazepin-oder Opiat-Entzug. Die Verbindungen können außerdem eine Anwendung in der Prävention der Toleranzentwicklung während der Langzeitbehandlung mit sedativen Arzneimitteln wie zum Beispiel Benzodiazepinen, Barbituraten und Morphin finden. Darüberhinaus können die Verbindungen als Anästhetika (Narkose), Anti-Schmerzmittel oder Antiemetika benutzt werden.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.
Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5 - 1000 mg, vorzugsweise 50 - 200 mg, wobei die Dosis als einmal zu verabreichende Einzeldosis oder unterteilt in zwei oder mehreren Tagesdosen gegeben werden kann.

Die Herstellung der erfindungsgemäßen Verbindungen erfolgt beispielsweise dadurch, daß man
a) eine Verbindung der allgemeinen Formel II 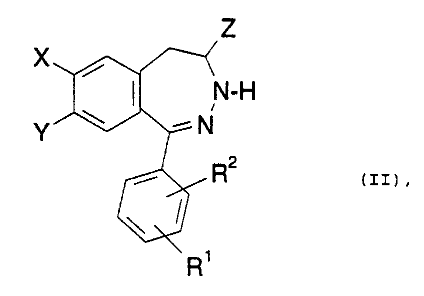 worin
   R¹, R², X und Y die obige Bedeutung haben, cyclisiert durch Umsetzung von
   α) Z = COOC₁₋₆-Alkyl mit R³-N=C=O zu Verbindungen mit A in der Bedeutung -CO-NR³-CO-
   β) Z = CH₂OH oder -CH₂-NHR³ mit Phosgen zu Verbindungen mit A in der Bedeutung - CH₂-O-CO- oder -CH₂-NR³-CO-
   γ) Z = -CH₂OH mit R³-CO-R⁴ zu Verbindungen mit A in der Bedeutung -CH₂-O-CR³R⁴, worin R³ und R⁴ die obige Bedeutung haben,
b) eine Verbindung der Formel III oder IV 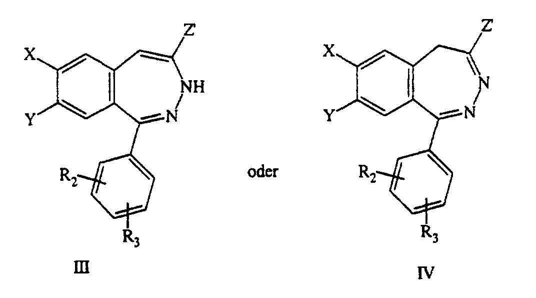 worin R¹, R², X und Y die obige Bedeutung haben, cyclisiert durch Umsetzung von
   α) Z' = -CH=CH-COOC₁₋₆-Alkyl mit Borantrimethylamin-Komplex und Bortrifluoridetherat zu Verbindungen mit A in der Bedeutung -(CH₂)₃- und -(CH₂)₂-CO-
   β) Z' = -CH=N-NH₂ in Gegenwart von Kupfersulfat zu Verbindungen mit A in der Bedeutung =CH-N=N-
   γ) Z' = -S-C₁₋₄-Alkyl mit Hydrazinhydrat und Säureanhydriden oder mit Säurehydraziden zu Verbindungen mit A in der Bedeutung =N-N=CR³-
   δ) Z' = -S-C₁₋₄-Alkyl mit α-Aminoacetalen zu Verbindungen mit A in der Bedeutung =N-CR³=CR⁴-
   ε) Z' = CH₂OH in CH₂NH₂ überführt, dieses acyliert und zu Verbindungen mit A in der Bedeutung =CH-N=CR³- cyclisiert,
c) eine Verbindung der Formel V, 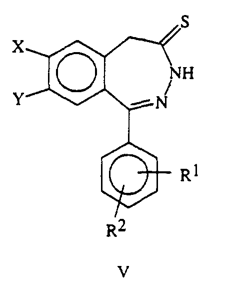 worin R¹, R², X und Y die oben gegebene Bedeutung haben, mit α-Aminoacetalen, α-Aminoketalen, H₂N-CH₂≡C-R³ oder mit Ammoniak und α-Halogenketonen umsetzt, und anschließend gewünschtenfalls die Nitrogruppe R¹ und/oder R² reduziert, die Aminogruppe acyliert oder alkyliert oder in Halogen oder Hydroxy oder Cyano überführt oder desaminiert oder X gleichzeitig mit der Reduktion der Nitro-Gruppe oder nacheinander enthalogeniert oder Wasserstoff durch Halogen substituiert oder Halogen austauscht gegen ein anderes Halogen, -PO₃R¹³R¹⁴, Cyano, C₁₋₆-Alkanoyl, C₁₋₆-Alkanoyloxy, Hydroxy, gegebenenfalls substituiertes C₂₋₆-Alkynyl, gegebenenfalls substituiertes C₂₋₆-Alkenyl, gegebenenfalls substituiertes C₁₋₆-Alkyl, C₁₋₆-Alkoxy, CF₃, C₁₋₆-Thioalkyl, COOR¹² oder Y umethert oder die Isomeren trennt oder die Salze bildet.

Die Ankondensation des Heterocyclus erfolgt zweckmäßigerweise an in 4-Stellung geeignet substituierten 2,3-Benzodiazepinen.

Die Umsetzung des Alkylrestes, worin Z = -COO-C₁₋₆-Alkyl bedeutet, mit R³-N=C=O in aprotischen Lösungsmitteln wie halogenierten Kohlenwasserstoffen bei Raumtemperatur oder erhöhter Temperatur führt zu Verbindungen der Formel I mit A in der Bedeutung von -CO-NR³-CO-. Werden Verbindungen der Formel II worin Z = -CH₂OH oder -CH₂-NHR³ bedeutet mit Phosgen in Gegenwart von tertiären Aminen in inerten Lösungsmitteln wie gegebenenfalls halogenierten Kohlenwasserstoffen umgesetzt, so erhält man Verbindungen der Formel I mit A in der Bedeutung -CH₂-O-CO- bzw. -CH₂-NR³-CO-.

Setzt man Verbindungen der Formel II, worin Z = -CH₂OH bedeutet, mit Carbonylverbindungen in Gegenwart von Säuren wie Salzsäure um, so erhält man als Cyclisierungsprodukt Verbindungen der Formel I, worin A -CH₂-O-CR³R⁴- bedeutet. Enthält das 2,3-Benzodiazepin in 4-Stellung eine Formylgruppe, so kann diese z.B. in einer Wittig-Reaktion in üblicher Weise in eine Verbindung der Formel III, worin Z' =-CH=CH-COO-C₁₋₆-Alkyl bedeutet, überführt werden.

Behandelt man den erhaltenen Acrylsäureester mit Borantrimethylaminkomplex und mit Bortrifluoridetherat in einem halogenierten Kohlenwasserstoff wie Dichlormethan, so erhält man Verbindungen der Formel I mit A = -(CH₂)₃- und -(CH₂)₂-CO-, die säulenchromatographisch getrennt werden können. Setzt man das in 4-Stellung formylierte 2,3-Benzodiazepin mit Hydrazinhydrat um, so erhält man das entsprechende Hydrazon-Derivat, das in polaren Lösungsmitteln gelöst und mit einer Lösung von Kupfersulfat in Wasser versetzt wird. Man erhält als Cyclisierungsprodukt Verbindungen der Formel I, worin A =CH-N=N- bedeutet.

Setzt man eine Verbindung der Formel III oder IV, worin Z' C₁₋₄-Alkyl-S- bedeutet, mit Säurehydraziden in Gegenwart einer Säure z.B. Sulfonsäure in einem organischen Lösungsmittel um, so erhält man Verbindungen der Formel I, worin A =N-N=CR³-bedeutet. Die Reaktion kann auch so durchgeführt werden, daß das Alkylthioderivat in einem organischen Lösungsmittel mit Hydrazinhydrat erhitzt wird, und anschließend mit einem Säureanhydrid zu dem gewünschten Produkt umgesetzt wird.

Erhitzt man das Methylthio-Benzodiazepin-Derivat mit α-Aminoacetalen H₂N-CR³H-CH-(O-Alkyl)₂, H₂N-CH₂-CR₄-(O-Alkyl)₂oder H₂N-CR³H-CR⁴-(O-Alkyl)₂ in Gegenwart einer Säure wie p-Toluolsulfonsäure, so erhält man Verbindungen der Formel I mit A in der Bedeutung =N-CR³=CH-, =N-CH=CR⁴- oder =N-CR³=CR⁴-.
Die gleichen Verbindungen der Formel I kann man herstellen, indem man eine Verbindung der Formel V mit dem entsprechenden α-Aminoacetal NH₂CHR³-CR⁴(OAlk)₂ gegebenenfalls in Lösungsmitteln wie Cellosolv® durch Einleiten von einem Inertgas wie z.B. Argon oder Stickstoff zum Entfernen des Schwefelwasserstoffes oder in Gegenwart von Schwefelfängern wie z.B. Quecksilberoxyd umsetzt. Unter dem Rest (OAlk)₂ sind entweder offene oder -gelegentlich vorteilhafter- auch cyclische Acetale oder Ketale zu verstehen. Verbindungen der Formel I können auch hergestellt werden, indem man nach literaturbekannten Verfahren Verbindungen der Formel V mit Propargylaminen H₂N-CH₂-C≡CR³ umsetzt (Eur. J. Med. Chem. 30, 429 (1995) oder Ann. Chem. 1987, (2), 103).

Verbindungen der Formel I erhält man auch, wenn man Verbindungen der Formel V mit Ammoniak in Lösungsmitteln wie Methanol oder Cellosolv® gegebenenfalls unter Druck oder unter Zugabe eines Schwefelfängers wie beispielsweise Silbertriflat oder Quecksilberoxid in das entsprechende Imin überführt und dann mit α-Halogenketonen umsetzt.

Ist Z' eine CH₂OH-Gruppe, so kann man den Alkohol in bekannter Weise durch Umsetzung nach Mitsunobu in das Azid oder in das Phtalimid überführen. Das Azid kann nach Literaturmethoden durch Reduktionsmittel oder durch Triphenylphosphin in das Amin überführt werden. Das Phtalimid kann durch Behandlung mit Hydrazin ebenfalls in das Amin überführt werden. Die Acylierung dieses Amins gelingt mit Säurechloriden oder -anhydriden nach bekannten Verfahren. Die anschliessende Cyclisierung mit Phosphoroxychlorid führt zu Verbindungen der Formel I mit der Bedeutung von A =CH-N=CR³-.

Die Reduktion in der Nitrogruppe wird in polaren Lösungsmitteln bei Raumtemperatur oder erhöhter Temperatur durchgeführt. Als Katalysatoren für die Reduktion sind Metalle wie Raney-Nickel oder Edelmetallkatalysatoren wie Palladium oder Platin oder auch Palladiumhydroxid gegebenenfalls auf Trägern geeignet. Statt Wasserstoff können auch zum Beispiel Ammoniumformiat, Cyclohexen oder Hydrazin in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfalls unter Zusatz eines Lösungsmittels wie zum Beispiel Wasser oder Methanol durchgeführt.

Wird eine Alkylierung einer Aminogruppe gewünscht, so kann nach üblichen Methoden - beispielsweise mit Alkylhalogeniden - oder nach der Mitsonubo Variante durch Umsetzung mit einem Alkohol in Gegenwart von Triphenylphosphin und Azodicarbonsäureester alkyliert werden, oder man unterwirft das Amin einer reduktiven Aminierung mit Aldehyden oder Ketonen gegebenenfalls nacheinander mit zwei verschiedenen Carbonylverbindungen, wobei man gemischte Derivate erhält [Literatur z.B. Verardo et al. Synthesis (1993), 121; Synthesis (1991), 447; Kawaguchi, Synthesis (1985), 701; Micovic et al. Synthesis (1991), 1043].

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin, nach der Schotten-Baumann-Variante in wäßriger Lösung bei schwach alkalischem pH-Wert oder durch Umsetzung mit einem Anhydrid in Eisessig.

Die Einführung der Halogene Chlor, Brom oder Jod über die Aminogruppe kann beispielsweise auch nach Sandmeyer erfolgen, indem man die mit Nitriten intermediär gebildeten Diazoniumsalze mit Kupfer(I)chlorid oder Kupfer(I)bromid in Gegenwart der entsprechenden Säure wie Salzsäure oder Bromwasserstoffsäure oder mit Kaliumjodid umsetzt. Statt der Diazoniumsalze können gegebenenfalls auch die Triazene eingesetzt werden. Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid. Die Entfernung der Aminogruppe kann entweder durch Umsetzung mit einem organischen Salpetrigsäureester in Tetrahydrofuran oder durch Diazotierung und reduktive Verkochung des Diazoniumsalzes beispielsweise mit phosphoriger Säure gegebenenfalls unter Zugabe von Kupfer (I) oxid bewerkstelligt werden.
Die Einführung von Fluor gelingt beispielsweise durch Balz Schiemann-Reaktion des Diazoniumtetrafluorborates oder nach J. Fluor. Chem. **76**,1996,59-62 durch Diazotierung in Gegenwart von HFxPyridin und anschliessende Verkochung gegebenenfalls in Gegenwart einer Fluoridionenquelle wie z.B. Tetrabutylammoniumfluorid..

Der Ersatz der Amino- durch die Hydroxygruppe erfolgt nach literaturbekannten Methoden vorzugsweise durch Überführung in das Triazen und anschliessende Behandlung mit einem stark sauren Ionenaustauscher (nach Tetr. Letters 1990, 4409).

Die Einführung von Halogenen in den annelierten Ring erfolgt nach literaturbekannten Verfahren z.B. durch Umsetzung mit N-Brom- oder N-Jodsuccinimid in polaren Lösungsmitteln wie Tetrahydrofuran, Acetonitril oder Dimethylformamid oder aber durch Umsetzung mit Jodsäure und Jod nach Lieb. Ann.Chem.**634**, 84, (1960).

Der Austausch eines Halogens im annelierten Ring erfolgt in literaturbekannter Weise gegebenenfalls unter Schwermetallkatalyse beispielsweise durch Palladium(II) oder Palladium(0) verbindungen durch zinnorganische- oder bororganische Verbindungen, C₂₋ ₆-Alkine, C₂₋₆-Alkene, Di- oder mono-alkylphosphit, Cyanid in Lösungsmitteln wie Toluol, Tetrahydrofuran oder Dimethylformamid (M. Kosugi et al. Chem. Lett. 7, 1225, 1984). Gegebenenfalls muss eine Base wie z.B. Triethylamin oder Natriumcarbonat und gegebenenfalls ein Cokatalysator wie z.B. Kupfer(I)jodid zugesetzt werden.

Halogen wie Brom oder Jod können auch mit Kupfersalzen wie Kupfer(I)cyanid (Einführung einer Nitrilgruppe), Kupferacetat (Einführung einer Alkanoyloxygruppe), Natriumalkoholat in Gegenwart von Kupfer(I)jodid (Einführung einer Alkoxygruppe) oder einer Mischung von Kupfer(I)jodid und Natriumtrifluoracetat (Einführung einer Trifluormethylgruppe)umgesetzt werden.

Das Halogen kann z.B. durch Umsetzung mit Butyllithium bei Temperaturen von 0°C bis -78°C in Lösungsmitteln wie Ether oder Tetrahydrofuran gegebenenfalls unter Zugabe komplexierender Agentien wie Tetramethylethylendiamin auch einem Halogen-Metallaustausch unterzogen und anschliessend in literaturbekannter Weise mit Elektrophilen wie beispielsweise Dimethylformamid, Alkylhalogeniden wie Jodiden oder Chloriden oder Aldehyden abgefangen werden.

Die Isomerengemische können nach den üblichen Methoden wie beispielsweise Kristallisation, Chromatographie oder Salzbildung in die Enantiomere aufgetrennt werden.

Die Herstellung der Salze erfolgt in üblicher Weise, indem man eine Lösung der Verbindung der Formel I mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder analog zu bekannten Verbindungen herstellbar.

Die Erfindung umfaßt auch die Verbindungen der Formel IIa und IIIa, deren Isomere und Salze 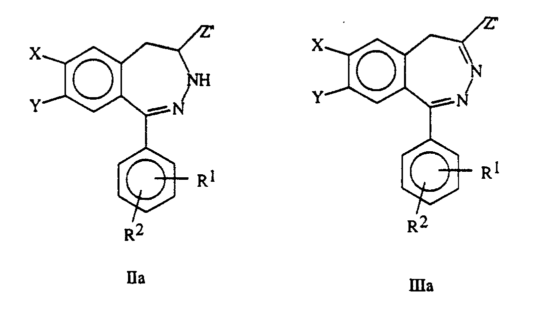 worin
R¹, R², X und Y die oben angegebene Bedeutung haben und
Z" -CH₂OH, -CHO, -COO-C₁₋₆-Alkyl, CH₂NHR³, 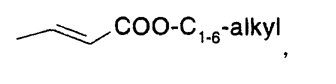 bedeutet und R³ die vorne genannte Bedeutung hat, die wertvolle Zwischenprodukte zur Herstellung pharmakologisch wirksamer Verbindungen darstellen. Die Umwandlung der Zwischenprodukte in Wirksubstanzen erfolgt nach den oben beschriebenen Verfahren.

Die Herstellung der Zwischenprodukte erfolgt nach bekannten oder hier beschriebenen Methoden. Enthält das 2,3-Benzodiazepin in 4-Stellung eine Methylgruppe, so kann diese beispielsweise mit SeO₂ zu Formyl oxidiert werden. Gewünschtenfalls kann die Formylgruppe reduziert werden zu -CH₂OH oder oxidiert werden zur Carboxylgruppe, die anschließend verestert werden kann oder die Formylgruppe kann überführt werden in - CH₂NHR³ oder einer Wittigreaktion unterworfen werden.

Die nachfolgenden Beispiele sollen das erfindungsgemäße Verfahren erläutern:

### Herstellung der Ausgangsverbindungen:

### I.) 8-Hydroxymethyl-7-methylcarbamoyl-5-(4-nitrophenyl)-8,9-dihydro-7H-1,3-dioxolo[4,5-h] [2,3]benzodiazepin

### A. 8-Formyl-5-(4-nitrophenyl)-9H-1,3-dioxolo[4,5-h][2,3]benzodiazepin

1,0 g (3,1 mmol) 8-Methyl-5-(4-nitrophenyl)-9H-1,3-dioxolo[4,5-h][2,3]benzodiazepin (French Patent No. 2566774) werden bei 90 °C in 15 ml DMF gelöst. Man fügt 0,38 g (3,4 mmol) SeO₂ hinzu und rührt 40 Minuten bei 90°C. Nach Abfiltration des Feststoffes wird das Produkt mit 100 ml Wasser ausgefällt, das Rohprodukt abfiltriert und mit Wasser gewaschen und getrocknet. Man erhält 1,04 g der Verbindung. Nach säulenchromatographischer Reinigung (Kieselgel, Elutionsmittel Benzol/Ethylacetat 20:1) und anschließender Suspension der kristallinen Verbindung in Ethanol erhält man 0,52 g (50%) Produkt. Schmelzpunkt 228 - 230 °C (Zersetzung).

### B. 8-Hydroxymethyl-5-(4-nitrophenyl)-8,9-dihydro-7H-1,3-dioxolo[4,5-h][2,3]-benzodiazepin

Eine Suspension von 7,0 g (20,7 mmol) des nach Reaktionsschritt A erhaltenen Aldehyds in 420 ml Ethanol wird unter Rühren auf 20 °C gekühlt und nach und nach mit 7,84 g (0,21 mol) NaBH₄ versetzt. Das Reaktionsgemisch wird 1 Stunde zum Sieden erhitzt, anschließend mit Aktivkohle versetzt und heiß filtriert. Das Lösungsmittel wird entfernt, der Rückstand in Dichlormethan aufgenommen, aufgearbeitet und man erhält 6,37 g (90%) Rohprodukt, das säulenchromatographisch an Kieselgel mit einem Gemisch von Benzol/Ethylacetat 1:1 als Elutionsmittel gereinigt wird. Man erhält 5,46 g (77%) reines Produkt mit dem Schmelzpunkt 132 - 134 °C.

### C. 8-Hydroxymethyl-7-methylcarbamoyl-5-(4-nitrophenyl)-8,9-dihydro-7H-1,3-dioxolo[4,5-h] [2,3]benzodiazepin

1,0 g (2,9 mmol) des nach Reaktionsschritt B erhaltenen Alkohols werden in 40 ml Dichlormethan gelöst und mit 0,5 ml (8,8 mmol) Methylisocyanat versetzt. Man läßt die Lösung 3 Tage bei Raumtemperatur stehen und engt anschließend ein. Der kristalline Rückstand wird in 10 ml Ethanol suspendiert und zum Sieden erhitzt. Man erhält 1,02 g (87%) gelbes Produkt mit dem Schmelzpunkt 242 - 243 °C (Zersetzung).

### II.) 5-(4-Aminophenyl)-8-hydroxymethyl-7-methylcarbamoyl-8,9-dihydro-7H-1,3-dioxolo[4,5-h] [2,3]benzodiazepin

Eine Suspension von 1,02 g (2,56 mmol) 8-Hydroxymethyl-7-methylcarbamoyl-5-(4-nitrophenyl)-8,9-dihydro-7H-1,3-dioxolo[4,5-h][2,3]benzodiazepin ( Stufe C) in 40 ml Ethanol wird unter Rühren mit 0,45 ml (9 mmol) 98%igem Hydrazin-Hydrat und RaNi-Katalysator versetzt. Nach 30 Minuten wird der Katalysator abfiltriert und die Lösung eingeengt. Der Rückstand wird in Ethanol umkristallisiert und man erhält 0,83 g (88%) Produkt mit dem Schmelzpunkt 136 - 138 °C.

### III.) 8-Hydroxymethyl-5-(4-nitrophenyl)-9H-1,3-dioxolo[4,5-h] [2,3]benzodiazepin

2,5g (7,41mmol) 8-Formyl-5-(4-nitrophenyl)-9H-1,3-dioxolo[4,5-h][2,3]benzodiazepin I,StufeA) werden in THF:Wasser=1:1 suspendiert und unter Rühren und Kühlen auf 20°C mit 0,14g (3,7mmol) Natriumboranat versetzt. Nach 45 min. Rühren wird filtriert und aus dem Filtrat mit 130ml Wasser das Rohprodukt gefällt. Man erhält 2,35g, die aus einem Gemisch aus 6,3ml Dimethylformamid und 1,3ml Wasser umkristallisiert werden. Man erhält 1,97g (78%) der Titelverbindung vom Schmelzpunkt 175°C (Zersetzung).

### Beispiel 1

### 9-Methyl-5-(4-nitrophenyl)-11H-1,3-dioxolo[4,5-h]imidazo[3,4-c][2,3]benzodiazepin-8.10(9H,10aH)-dion

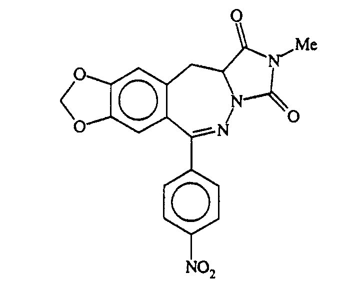

### A. 5-(4-Nitrophenyl)-9H-[1,3-]dioxolo[4,5-h][2,3]-benzodiazepin-8-carbonsäure

Zu einer Lösung von 3,0 g AgNO₃ in Wasser (50 ml) werden 42 ml einer 4%igen NaOH-Lösung gegeben. Zu der heterogenen Mischung wird langsam die Lösung von 3,0 g 8-Formyl-5-(4-nitrophenyl)-9H-1,3-dioxolo[4,5-h-][2,3]-benzodiazepin in 120 ml Dioxan gegeben und das Reaktionsgemisch 30 Minuten bei 25 °C gerührt. Nach Zugabe von Aktivkohle wird filtriert, das Filtrat im Vakuum bei 50 - 60 °C eingeengt, die entstandene Suspension mit 30 ml Wasser verdünnt und abgekühlt. Nach Stehen über Nacht wird abfiltriert, der Niederschlag mit 2 x 10 ml Eiswasser gewaschen und man erhält 1,94 g des Natriumsalzes, das in 60 ml heißen Wasser gelöst und nach dem Abkühlen mit 1 ml Essigsäure angesäuert wird. Nach Filtration und Waschen mit Wasser erhält man 1,66 g (53%) Produkt mit dem Schmelzpunkt 196 - 198 °C (Zersetzung).

### B. 8-(Methoxycarbonyl)-5-(4-nitrophenyl)-9H-1,3-dioxolo[4,5-h][2,3]benzodiazepin

8,0 g (22,0 mmol) der nach Reaktionsschritt A erhaltenen Verbindung werden in 390 ml Methanol suspendiert und nach Addition von 4,2 ml (34,4 mmol) Bortrifluoridetherat wird die Mischung 3 Stunden zum Rückfluss erhitzt. Das Lösungsmittel wird abgezogen, der Rückstand in Dichlormethan und 100 ml einer 10%igen Na₂CO₃-Lösung aufgenommen und 30 Minuten gerührt. Das nach Aufarbeitung¹ erhaltene Rohprodukt wird säulenchromatographisch gereinigt (Kieselgel, Eluent : Benzol/Ethylacetat 20:1) und man erhält 3,86 g (46%) der Titelverbindung mit dem Schmelzpunkt 235 - 238 °C (Zersetzung).
¹ Aufarbeitung meint hier und bei weiteren Beispielen: Die organische Phase wird mit Wasser gewaschen, getrocknet, filtriert und eingeengt.

### C. 8-(Methoxycarbonyl)-5-(4-nitrophenyl)-8,9-dihydro-7H-1,3-dioxolo[4,5-h][2,3]-benzodiazepin

Eine Suspension von 2,94 g (8,0 mmol) der nach Reaktionsschritt B erhaltenen Verbindung in 30 ml Dichlormethan wird unter Rühren mit 15 ml Trifluoressigsäure versetzt. 6,3 ml (40 mmol) Triethylsilan werden hinzugefügt und 24 Stunden bei Raumtemperatur gerührt. Anschließend wird mit 30 ml Dichlormethan versetzt und unter Rühren und Kühlen mit Eiswasser eine Lösung von 12,3 g Na₂CO₃ in 60 ml Wasser hinzugefügt. Der nach Aufarbeitung erhaltene Rückstand wird mit 20 ml Methanol behandelt und man erhält 2,85 g (96 %) der Titelverbindung mit dem Schmelzpunkt 161 - 164 °C.

### D. 9-Methyl-5-(4-nitrophenyl)-11H-1,3-dioxolo[4,5-h]imidazo[3,4-c][2,3]benzodiazepin-8,10(9H,10aH)-dion

Man löst 0,85 g (2,3 mmol) der nach Reaktionsschritt C erhaltenen Verbindung in 30 ml Dichlormethan und läßt 3 Wochen bei Raumtemperatur mit 1,0 ml (17,5 mmol) Methylisocyanat reagieren. Das Lösungsmittel wird abgezogen und der Rückstand durch Kochen mit Methanol gereinigt. Man erhält nach Absaugen 0, 77 g (84%) des Produktes mit dem Schmelzpunkt 242 - 244 °C (Zersetzung).

### Beispiel 2

### 5-(4-Aminophenyl)-9-methyl-11H-1,3-dioxolo[4,5-h]imidazo[3,4-c][2,3]benzodiazepin-8,10(9H,10aH)-dion

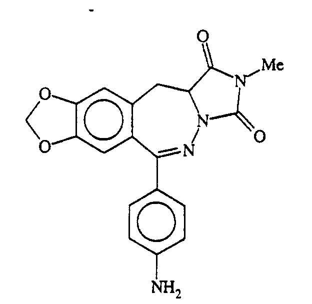

0,61 g (1,55 mmol) der nach Beispiel 1 erhaltenen Nitro-Verbindung werden mit RaNi/Hydrazin-Hydrat in einer Mischung von Dichlormethan/Methanol 2:1 reduziert. Der Katalysator wird abfiltriert, die Lösung eingeengt, der kristalline Rückstand mit Wasser gewaschen und man erhält 0,54g (54%) der Titelverbindung mit dem Schmelzpunkt > 270 °C (Zersetzung).

### Beispiel 3

### 9-Methyl-5-(4-nitrophenyl)-9,10,10a,11-tetrahydro-8H-1,3-dioxolo[4,5-i]imidazo[3,4-c][2,3]benzodiazepin-8-on

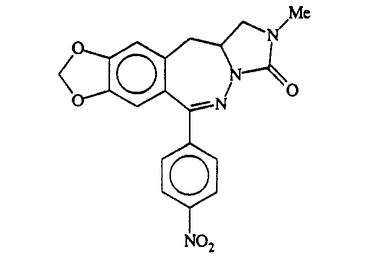

### A. 8-[(Methylimino)methyl]-5-(4-nitrophenyl)-9H-1,3-dioxolo[4,5-h][2,3]benzodiazepin

2,0 g (5,9 mmol) 8-Formyl-5-(4-nitrophenyl)-9H-1,3-dioxolo[4,5-h][2,3]benzodiazepin werden in 100 ml einer Mischung von Dichlormethan - Methanol 1 : 1 gelöst, mit 40 ml einer 33%igen Lösung von Methylamin in Ethanol versetzt und 24 Stunden bei Raumtemperatur stehengelassen. Anschließend wird das Lösungsmittel abgezogen und der Rückstand mit 25 ml Ethanol gekocht. Nach Filtrieren erhält man 1,95 g (93%) der Titelverbindung mit dem Schmelzpunkt 245 - 247 °C (Zersetzung).

### B.

5,5 g (15,7 mmol) des nach Reaktionsschritt A erhaltenen Imins werden in 800 ml Ethanol suspendiert und portionsweise unter Rühren mit 26 ml konz. HCl versetzt. Zu der erhaltenen Lösung gibt man 13,6 g (0,36 mmol) NaBH₄ über 1 Stunde in kleinen Portionen. Man rührt 30 Minuten nach, filtriert, engt das Filtrat ein und reinigt den Rückstand säulenchromatographisch mit Methanol als Elutionsmittel. Man erhält 3,67 g (66%) 8-[(Methylamino)methyl]-5-(4-nitrophenyl)-9H-1,3-dioxolo[4,5-h][2,3]benzodiazepin mit dem Schmelzpunkt 110 - 112 °C.

### C. 9-Methyl-5-(4-nitrophenyl)-9,10,10a,11-tetrahydro-8H-1,3-dioxolo[4,5-h]imidazo[3,4-c][2,3]benzodiazepin-8-on

Zu einer gekühlten und gerührten Lösung von 1,05 g (3,0 mmol) der Methylaminomethyl-Verbindung nach Reaktionsschritt B und 0,99 ml (7,2 mmol) Triethylamin in 15 ml Dichlormethan werden 2,26 ml (3,6 mmol) einer 15,6%igen Phosgen-Lösung in Toluol tropfenweise hinzugefügt. Die Mischung wird 2 Stunden gerührt und zur Trockne eingeengt. Der Rückstand wird mit Wasser behandelt und man erhält 1,08 g Rohprodukt, das durch Kochen in 10ml Ethanol gereinigt wird. Nach Absaugen erhält man 0,96 g (84%) der Titelverbindung mit dem Schmelzpunkt 252 - 255 °C.

### Beispiel 4

### 5-(4-Aminophenyl)-9-methyl-9,10,10a,11-tetrahydro-8H-1,3-dioxolo[4,5-h]imidazo[3,4-c][2,3]benzodiazepin-8-on

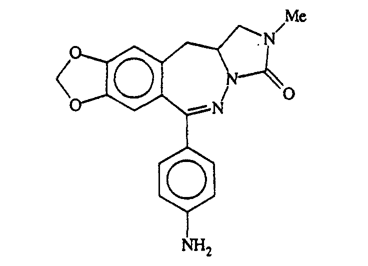

0,38 g (1,0 mmol) der Nitroverbindung des Beispiels 3 werden in 20 ml Methanol mit RaNi und Hydrazin-Hydrat analog Beispiel 2 reduziert. Nach Kochen mit Ethanol erhält man 0,25g (71%) der Titelverbindung aus Ethanol mit dem Schmelzpunkt 280 - 289 °C (Zersetzung).

### Beispiel 5

### 5-(4-Nitrophenyl)-9,10,10a,11-tetrahydro-8H-1,3-dioxolo[4,5-h]pyrrolo[1,2-c][2,3]benzodiazepin

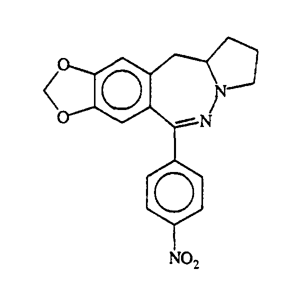

### A.

Methyl-3-[5-(4-nitrophenyl)-9H-1,3-dioxolo[4,5-h][2,3]benzodiazepin-8-yl]-propenolat Zu einer Lösung von 4,0 g (11,8 mmol) 8-Formyl-5-(4-nitrophenyl)-9H-1,3-dioxolo[4,5-h][2,3]benzodiazepin in 200 ml einer Mischung aus Dichlormethan und Methanol=1:1 werden nacheinander unter Rühren 1,9 ml (13,6 mmol) Triethylamin und 5,64 g (13,6 mmol) Methoxycarbonylmethyl-triphenylphosphoniumbromid gegeben. Nach 2 Stunden bei Raumtemperatur wird das Lösungsmittel der Suspension abgezogen, der Rückstand in 70 ml Ethanol suspendiert und filtriert. Nach je dreimaligem Waschen mit 10ml Ethanol und 50ml Wasser und anschlieessenden Trocknen des abgesaugten Feststoffes erhält man 4,44 g (95%) Methyl-3-[5-(4-nitrophenyl)-9H-1,3-dioxolo[4,5-h][2,3]benzodiazepin-8-yl]-propenolat mit dem Schmelzpunkt >260 °C.

### B. 5-(4-Nitrophenyl)-9,10,10a,11-tetrahydro-8H-1,3-dioxolo[4,5-h]pyrrolo[1,2-c][2,3]benzodiazepin

Zu der Suspension von 9,0 g (22,9 mmol) der nach Stufe A erhaltenen Verbindung in trockenem Dichlormethan (660 ml) werden 2,22 g (30,4 mmol) Borantrimethylamin-Komplex gegeben und unter starkem Rühren tropfenweise 5,0 ml (40,6 mmol) Bortrifluoridetherat hinzugefügt. Nachdem über Nacht gerührt wurde, versetzt man mit 300 ml 10%iger Na₂CO₃-Lösung, trennt die organische Phase ab und arbeitet in üblicher Weise auf. Das Rückstand wird säulenchromatographisch gereinigt (Kieselgel, Elutionsmittel Benzol:Ethylacetat=4 : 0,5). Man erhält 0,72 g (9%)der Titelverbindung mit dem Schmelzpunkt 170 - 172°C (Zersetzung).

### Beispiel 6

### 5-(4-Nitrophenyl-)-9,10,10a,11-tetrahydro-8H-1,3-dioxolo[4,5-h]pyrrolo[1,2-c][2,3]benzodiazepin[4,5-h]8-on

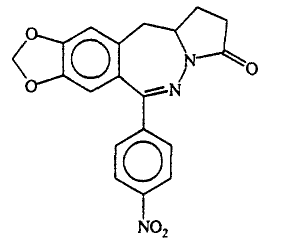

Wird das nach Beispiel 5 erhaltene Rohprodukt an Kieselgel mit der polareren Mischung Ethylacetat:Benzol=4:1 als Elutionsmittel chromatographiert, erhält man 1,26 g (15%) der Titelverbindung mit dem Schmelzpunkt 251 - 253 °C (Zersetzung).

### Beispiel 7

### 5-(4-Aminophenyl)-9,10,10a,11-tetrahydro-8H-1,3-dioxolo[4,5-h]pyrrolo[1,2-c][2,3]benzodiazepin

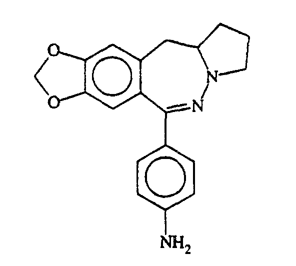

0,68 g (1,94 mmol) der Nitroverbindung nach Beispiel 5 wird in Methanol mit RaNi/Hydrazinhydrat analog Beispiel 2 reduziert. Man erhält nach Umkristallisation aus einem Gemisch von 50% Ethanol/Wasser 0,48 g (77%) der Titelverbindung mit dem Schmelzpunkt 153 - 155 °C.

### Beispiel 8

### 5-(4-Aminophenyl)-9,10,10a,11-tetrahydro-8H-1,3-dioxolo[4,5-h]pyrrolo[1,2-c][2,3lbenzodiazepin[4,5-h]8-on

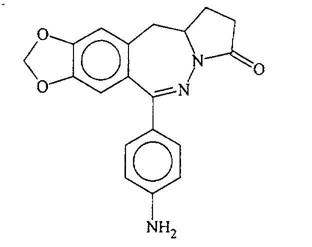

Die nach Beispiel 6 erhaltene Verbindung wird in Dichlormethan/Methanol 1 : 1 RaNi/Hydrazinhydrat reduziert analog Beispiel 2. Nach Umkristallisation aus Ethanol erhält man 0,8 g (80%) der Titelverbindung mit dem Schmelzpunkt 291 - 292 °C (Zersetzung).

### Beispiel 9

### 5-(4-Nitrophenyl)-11H-1,3-dioxolo[4,5-i][1,2,3]triazolo[4,3-c][2,3]benzodiazepin

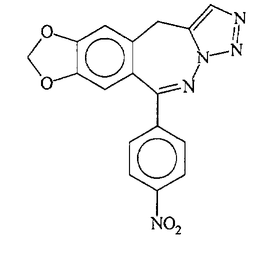

### A. 8-Formyl-5-(4-nitrophenyl)-9H-1,3-dioxolo[4,5-h][2,3]benzodiazepin-hydrazon

3,0 g (8,9 mmol) 8-Formyl-4-(4-nitrophenyl)-9H-1,3-dioxolo[4,5-h][2,3]benzodiazepin werden in 18 ml DMF suspendiert und mit 1,3 ml (26,7 mmol) 98% Hydrazinhydrat versetzt und 1 Stunde auf 110 - 120 °C erwärmt. Nach dem Abkühlen auf Raumtemperatur wird in Wasser gegeben, abgesaugt und der Rückstand mit Wasser gewaschen und getrocknet. Dieses Rohprodukt wird aus DMF - Wasser umkristallisiert 80% . Man erhält 2,51 g (80%) der Titelverbindung mit dem Schmelzpunkt 221 - 223 °C (Zersetzung).

### B. 5-(4-Nitrophenyl)-11H-1,3-dioxolo[4,5-i][1,2,3]triazolo [4,3-c][2,3]benzodiazepin

2,0 g (5,7 mmol) der nach Verfahrensschritt A erhaltenen Verbindung werden unter schwachem Erwärmen in THF - Methanol 1 : 1 gelöst und unter Rühren mit einer Lösung von 7,0 g CuSO₄ · 5H₂O in 190 ml Wasser versetzt. Nach 30 Minuten wird das organische Lösungsmittel entfernt und der Rückstand in Chloroform und Wasser gelöst. Die organische Phase wird abgetrennt, mit Wasser gewaschen, getrocknet und eingeengt. Man erhält nach Umkristallisation aus einer Mischung von DMF und Wasser 10 : 11,33 g (67%) der Titelverbindung mit dem Schmelzpunkt 257 - 258 °C (Zersetzung).

### Beispiel 10

### 5-(4-Aminophenyl)-11H-1,3-dioxolo[4,5-i][1,2,3]triazolo [4,3-c][2,3]benzodiazepin

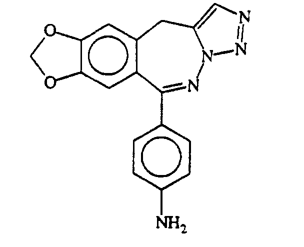

1,17 g (3,35 mmol) der Nitroverbindung des Beispiels 9 werden in 100 ml einer Mischung Dichlormethan-Methanol 1 : 1 mit RaNi/Hydrazinhydrat reduziert analog Beispiel 2. Man erhält nach Umkristallisation aus DMF - Wasser (10:1) 0,8 g (74%) der Titelverbindung mit dem Schmelzpunkt > 260 °C (Zers.).

### Beispiel 11

### 10a, 11-Dihydro-8,8-Dimethyl-5-(4-nitrophenyl)-10H-1,3-dioxolo[4,5-h]oxazolo-[3,4-c][2.3]benzodiazepin

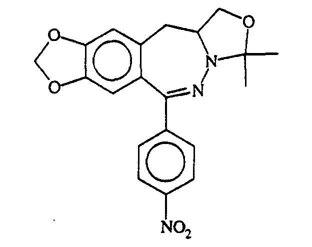

1,87 g (5,48 mmol) 8-Hydroxymethyl-5-(4-nitrophenyl)-8,9-dihydro-7H-1,3-dioxolo[4,5-h][2,3]benzodiazepin werden in 25 ml Aceton gelöst und mit 0,54 ml (6,67 mmol) 37%iger HCl versetzt. Nach 30 Minuten wird das Reaktionsgemisch abgekühlt und das Hydrochlorid abfiltriert. Die Dichlormethan-Suspension dieses Salzes wird mit 20 ml 8%iger NaHCO₃-Lösung geschüttelt bis das Salz gelöst ist. Die organische Phase wird abgetrennt, mit Wasser gewaschen, getrocknet und eingeengt. Man erhält nach Umkristallisation 1,7 g (81%) der Titelverbindung. Schmelzpunkt 171 - 173 °C.

### Beispiel 12

### 5-(4-Aminophenyl)-10a,11-Dihydro-8,8-Dimethyl-10H-1,3-dioxolo[4,5-h]oxazolo-[3,4-c][2,3]benzodiazepin

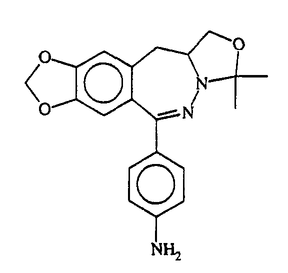

Analog Beispiel 2 erhält man aus 1,7 g (4,46 mmol) der Verbindung des Beispiels 11 mit RaNi/Hydrazinhydrat in Methanol 1,2 g (76%) der Titelverbindung mit dem Schmelzpunkt 133 - 135 °C (Ethanol:Wasser= 1: 1).

### Beispiel 13

### 10a,11-Dihydro-5-(4-nitrophenyl)-10H-1,3-dioxolo[4,5-h]oxazolo-[3,4-c][2,3]benzodiazepin-8-on

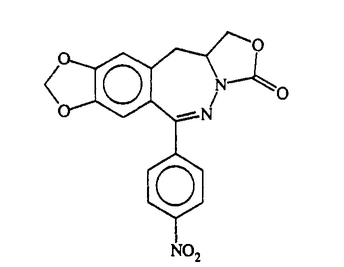

Unter Rühren und Kühlen wird eine Lösung von 1,0 g (2,93 mmol) 8-Hydroxymethyl-5-(4-nitrophenyl)-8,9-dihydro-7H-1,3-dioxolo [4,5-h][2,3]-benzodiazepin nacheinander mit 0,98 ml (7,03 mmol) Triethylamin und 2,23 ml (3,52 mmol) einer 15,6%igen Phosgen-Lösung in Toluol versetzt. Danach wird die Reaktionsmischung 3 Stunden auf 25 °C erwärmt. Der Niederschlag des Rohproduktes wird abgetrennt, das Filtrat eingeengt und der Rückstand mit Wasser behandelt. Die vereinigten Rückstände werden mit Ethanol gekocht und man erhält nach Absaugen 0,72 g (67%) der Titelverbindung mit dem Schmelzpunkt > 250 °C (Zersetzung).

### Beispiel 14

### 5-(4-Aminophenyl)-10a,11-dihydro-10H-1,3-dioxolo[4,5-h]oxazolo-[3,4-c][2,3]benzodiazepin-8-on

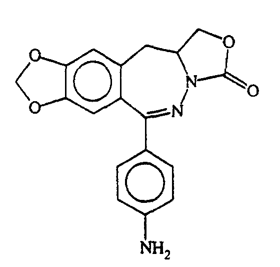

0,5 g (1,36 mmol) der nach Beispiel 13 erhaltenen Nitroverbindung werden in DMF mit RaNi/Hydrazinhydrat analog Beispiel 2 reduziert. Das Rohprodukt wird in 4,5ml heissem Ethanol gereinigt und man erhält 0,49 g (85%) der Titelverbindung mit dem Schmelzpunkt 173 - 175°C.

### Beispiel 15

### 5-(4-Nitrophenyl)-8-methyl-11H-1,3-dioxolo[4,5-h][1,2,4]triazolo[4,3-c][2.3]benzodiazepin

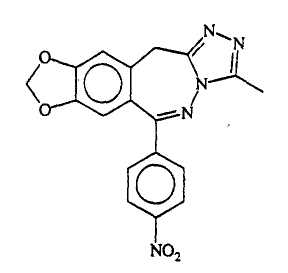

### A. 5-(4-Nitrophenyl)-1,3-dioxolo[4,5-g]-isochroman

36,0 g (0,21 mmol) 2-(1,3-Benzodioxol-5-yl)-ethanol werden mit einer equivalenten Menge 4-Nitrobenzaldehyd analog C.A. 105, 1986, 226357, umgesetzt und man erhält 50,7 g (81%) Titelverbindung. Schmelzpunkt 149 - 150 °C (Ethanol).

### B. 4,5-Methylendioxy-2-(4-nitrobenzoyl)-phenylessigsäure

10,0 g (33,4 mmol) des nach Reaktionsschritt A erhaltenen Isochromans werden nach Jones zur Titelverbindung oxidiert (F. Gatta et al. Il Farmaco 40, 1985, 942 - 955) Ausbeute 46%, Schmelzpunkt 237 - 239 °C (Methylcellosolve ®).

### C. 5-(4-Nitrophenyl)-7H-1,3-dioxolo[4,5-h] [2,3]benzodiazepin-8(9H)-on

5,0 g (15,2 mmol) der nach Reaktionsschritt B erhaltenen Verbindung werden in Methylcellosolve ® (50 ml) bei 110 °C mit 5,0 ml 98% Hydrazinhydrat 2,5 Stunden umgesetzt. Das Lösungsmittel wird im Vakuum abgezogen, der Rückstand in Dichlormethan (200 ml) und 40%iger Essigsäure (20 ml) gelöst. Die organische Phase wird abgetrennt, mit Wasser gewaschen und getrocknet. Man fügt 4,0 g (19,4 mmol) 1,3-Dicyclohexylcarbodiimid zu und läßt über Nacht bei Raumtemperatur stehen. Der Niederschlag wird abfiltriert, das Filtrat eingeengt. Beide Feststoffe werden mit 60ml Ethanol erhitzt und dann abgesaugt. Man erhält 1,95 g (39%) der Titelverbindung, Schmelzpunkt 292 - 294 °C.

### D. 5-(4-Nitrophenyl)-7H-1,3-dioxolo[4,5-h][2,3]benzodiazepin-8(9H)-thion

6,5 g (20,0 mmol) der nach Reaktionsschritt C erhaltenen Verbindung und 7,6 g (30,0 mmol) Phosphorpentasulfid werden in 100 ml Pyridin auf 80 °C erhitzt. Nach 1,5 Stunden wird das Reaktionsgemisch in 400 ml Wasser gegossen und der pH-Wert der Lösung mit Essigsäure auf 6,5 eingestellt. Der Niederschlag wird abfiltriert, gewaschen und getrocknet. Man erhält 4,36 g (64%) Produkt, Schmelzpunkt 257 - 258 °C (Aceton).

In analoger Weise werden über die Stufen **A-D** hergestellt:
5-(4-Chlorphenyl)-7H-1,3-dioxolo[4,5-h][2,3]benzodiazepin-8(9H)-thion
5-(4-Fluorphenyl)-7H-1,3-dioxolo[4,5-h][2,3]benzodiazepin-8(9H)-thion
5-(2-Fluorphenyl)-7H-1,3-dioxolo[4,5-h] [2,3]benzodiazepin-8(9H)-thion
5-(3-Chlorphenyl)-7H-1,3-dioxolo[4,5-h][2,3]benzodiazepin-8(9H)-thion
5-(2-Chlorphenyl)-7H-1,3-dioxolo[4,5-h][2,3]benzodiazepin-8(9H)-thion
5-Phenyl-7H-1,3-dioxolo[4,5-h][2,3]benzodiazepin-8(9H)-thion

### E. 8-(Methylthio)-5-(4-nitrophenyl)-9H-1,3-dioxolo-[4,5-h][2,3]benzodiazepin

2,2 g (6,45 mmol) der nach Reaktionsschritt D erhaltenen Verbindung werden in 500 ml Aceton gelöst und mit 2,22 g K₂CO₃ und 2 ml (32mmol) Methyliodid versetzt. Man rührt ca. 2 Tage, gießt in Wasser, trennt den Niederschlag ab und wäscht ihn mit Wasser. Nach Trocknen erhält man 2,0 g (87%) Produkt, Schmelzpunkt 280 - 281 °C.

Analog werden hergestellt:
5-(2-Fluorphenyl)-8-(methylthio)-9H-1,3-dioxolo-[4,5-h][2,3]benzodiazepin
5-(3-Chlorphenyl)-8-(methylthio)-9H-1,3-dioxolo-[4,5-h][2,3]benzodiazepin
5-(2-Chlorphenyl)-8-(methylthio)-9H-1,3-dioxolo-[4,5-h][2,3]benzodiazepin
8-(Methylthio)-5-phenyl-9H-1,3-dioxolo-[4,5-h][2,3]benzodiazepin

### F. 5-(4-Nitrophenyl)-8-methyl-11H-1,3-dioxolo[4,5-h][1,2,4]triazolo[4,3-c][2,3]benzodiazepin

0,53 g (1,50 mmol) der nach Reaktionsschritt E erhaltenen Verbindung, 0,22g (3,0 mmol) Essigsäurehydrazid und 0,1 g p-Toluolsulfonsäure werden in 25 ml Methylcellosolve ® unter Rühren auf 120 °C erwärmt. Nach 45 Minuten wird die Reaktionsmischung in Wasser gegossen, der Niederschlag abgetrennt, mit Wasser gewaschen und getrocknet. Man erhält 0,45 g (83%) Produkt, Schmelzpunkt 292 - 294 °C (Zersetzung).

### Beispiel 16

### 5-(4-Aminophenyl)-8-methyl-11H-1,3-dioxolo[4,5-h][1,2,4]triazolo[4,3-c][2,3]benzodiazepin

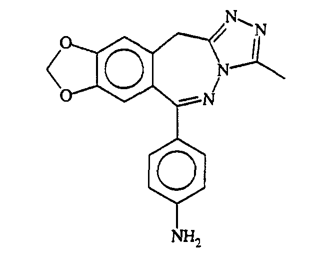

0,45 g der nach Beispiel 15 erhaltenen Nitro-Verbindung werden in Methanol analog Beispiel 2 mit RaNi/Hydrazinhydrat reduziert und man erhält 0,40 g (97%) Produkt, Schmelzpunkt 278 - 280 °C (Ethanol).

### Beispiel 17

### 5-(4-Nitrophenyl)-8-ethyl-11H-1,3-dioxolo[4,5-h][1,2,4]triazolo[4,3-c][2,3,benzodiazepin

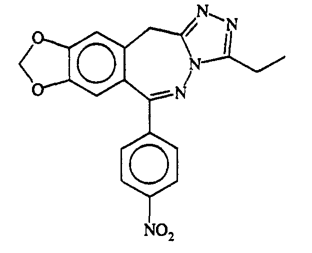

Analog Beispiel 15 erhält man nach Verfahrensschritt F mit Propionsäurehydrazid das Produkt. Ausbeute 71%, Schmelzpunkt 234 - 235 °C.

Grundsätzlich analog werden hergestellt:
5-(4-Nitrophenyl)-8-propyl-11H-1,3-dioxolo[4,5-h][1,2,4]triazolo[4,3-c][2,3]benzodiazepin (Schmpkt. 124-126°C)
5-(4-Nitrophenyl)-8-cyclopropyl-11H-1,3-dioxolo[4,5-h][[1,2,4]triazolo(4,3-c][2,3]benzodiazepin (Schmpkt. 154-156°C)
5-(4-Nitrophenyl)-8-n-butyl-11H-1,3-dioxolo[4,5-h][1,2,4]triazolo[4,3-c][2,3]benzodiazepin (Schmpkt. 124-125°C)
5-(4-Nitrophenyl)-8-methoxymethyl-11H-1,3-dioxolo[4,5-h][1,2,4]triazolo[4,3-c][2,3]benzodiazepin (Schmpkt. 142-143°C)
5-(2-Chlorphenyl)-8-methyl-11H-1,3-dioxolo[4,5-h][1,2,4]triazolo[4,3-c][2,3]benzodiazepin
5-(3-Chlorphenyl)-8-(methyl)-11H-1,3-dioxolo[4,5-h] [1,2,4]triazolo[4,3-c] [2,3]benzodiazepin
5-(2-Fluorphenyl)-8-(methyl)-11H-1,3-dioxolo[4,5-h][1,2,4]triazolo[4,3-c][2,3]benzodiazepin
8-Methyl-5-phenyl-11H-1,3-dioxolo[4,5-h][1,2,4]triazolo[4,3-c][2,3]benzodiazepin 8-Ethyl-5-phenyl-11H-1,3-dioxolo[4,5-h][1,2,4]triazoIo[4,3-c][2,3]benzodiazepin
8-Cyclopropyl-5-phenyl-11H-1,3-dioxolo[4,5-h](1,2,4]triazolo(4,3-c][2,3]benzodiazepin
8-(4-Nitrophenyl)-5-phenyl-11H-1,3-dioxolo[4,5-h][1,2,4]triazolo[4,3-c] [2,3]benzodiazepin

### Beispiel 18

### 5-(4-Aminophenyl)-8-ethyl-11H-1,3-dioxolo[4,5-h][1,2,4]triazolo[4,3-c][2,3]benzodiazepin

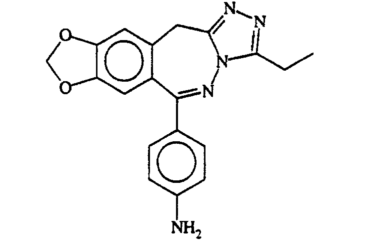

Man reduziert die nach Beispiel 17 erhaltene Nitro-Verbindung analog Beispiel 16. Ausbeute 84 %, Schmelzpunkt 265 - 266 °C (Ethanol).

Analog werden hergestellt:
5-(4-Aminophenyl)-8-propyl-11H-1,3-dioxolo[4,5-h][1,2,4]triazolo[4,3-c][2,3]benzodiazepin (Schmpkt. 202-203°C, Essigester)
5-(4-Aminophenyl)-8-cyclopropyl-11H-1,3-dioxolo[4,5-h][1,2,4]triazolo[4,3-c][2,3]benzodiazepin (Schmpkt. 191-192°C, Essigester/Diethylether)
5-(4-Aminophenyl)-8-n-butyl-11H-1,3-dioxolo[4,5-h][1,2,4]triazolo[4,3-c][2,3]benzodiazepin (Schmpkt. 186-187°C, Essigester)
5-(4-Aminophenyl)-8-methoxymethyl-11H-1,3-dioxolo[4,5-h][1,2,4]triazolo[4,3-c][2,3]benzodiazepin (Schmpkt. 261-263°C, Ethanol)
8-(4-Aminophenyl)-5-phenyl-11H-1,3-dioxolo[4,5-h][1,2,4]triazolo[4,3-c] [2,3]benzodiazepin

### Beispiel 19

### 5-(4-Nitrophenyl)-8-(4-pyridyl)-11H-1,3-dioxolo[4,5-h][1,2,4]triazolo[4,3-c][2,3]benzodiazepin

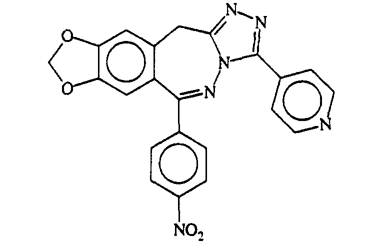

Das bei Beispiel 15E beschriebene Methylthioderivat wird analog Beispiel 15F mit Isonikotinsäurehydrazid in DMF und konz. Salzsäure als Katalysator umgesetzt. Ausbeute 76%, Schmelzpunkt 305 - 308 °C (Zersetzung).

Analog wird hergestellt
5-Phenyl-8-(4-pyridyl)-11H-1,3-dioxolo[4,5-h][1,2,4]triazolo[4,3-c][2,3]benzodiazepin

### Beispiel 20

### 5-(4-Aminophenyl)-8-(4-pyridyl)-11H-1,3-dioxolo[4,5-h][1,2,4]triazolo[4,3-c][2,3]benzodiazepin

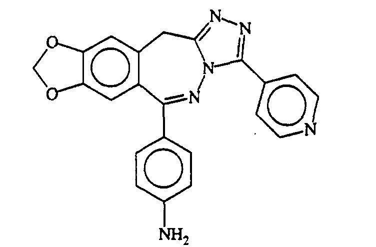

Man reduziert die Nitroverbindung des Beispiels 19 analog Beispiel 2, Ausbeute 46 %, Schmelzpunkt 301 - 302 °C (Zersetzung).

### Beispiel 21

### 5-(4-Nitrophenyl)-11H-1,3-dioxolo[4,5-h][1,2,4]triazolo[4,3-c][2,3 ]benzodiazepin

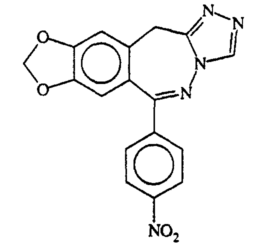

0,53 g der nach Beispiel 15E erhaltenen Methylthio-Verbindung werden analog Beispiel 15F mit 0,18 g Ameisensäurehydrazid in DMF mit konz. HCl als Katalysator umgesetzt. Nach Chromatographie über Kieselgel mit Chloroform:Methanol=95:5 als Elutionsmittel erhält man 0,34 g (71 %) Produkt. Schmelzpunkt 182 - 183 °C.

Analog dazu wird hergestellt:
5-Phenyl-11H-1,3-dioxolo[4,5-h][1,2,4]triazolo[4,3-c][2,3]benzodiazepin

### Beispiel 22

### 5-(4-Aminophenyl)-11H-1,3-dioxolo[4,5-h][1,2,4]triazolo[4,3-c][2,3]benzodiazepin

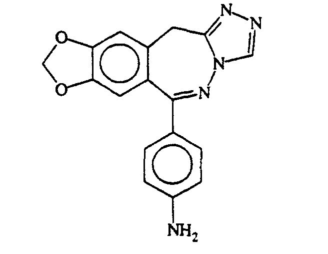

Die nach Beispiel 21 erhaltene Nitro-Verbindung wird analog Beispiel 2 reduziert. Ausbeute 70%. Schmelzpunkt 280 - 281 °C (Ethanol).

### Beispiel 23

### 5-(4-Nitrophenyl-8-(trifluormethyl)-11H-1,3-dioxolo[4,5-h][1,2,4]triazolo[4,3-c][2,3]benzodiazepin

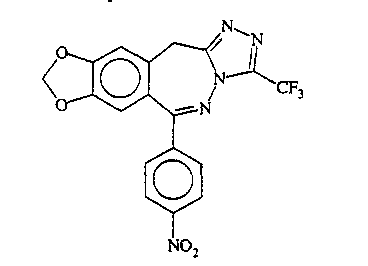

0,53 g (1,50 mmol) der in Beispiel 15E erhaltenen Methylthioverbindung werden mit 1,5 ml Hydrazinhydrat in 25 ml Methylcellosolve® zum Sieden erhitzt. Nach 1 Stunde wird das Lösungsmittel abgezogen, der Rückstand in Wasser aufgenommen und der Niederschlag abfiltriert. Nach dem Trocknen wird die Verbindung in Dichlormethan gelöst und unter Rühren und Kühlen mit Eiswasser tropfenweise mit 0,40 ml Trifluoressigsäureanhydrid versetzt. Die Reaktionsmischung wird dann 1 Stunde zum Sieden erhitzt und anschließed zur Trockene eingeengt. Der Rückstand wird in Toluol aufgenommen, 20Mmn erhitzt und das Lösungsmittel anschließend entfernt. Nach Chromatographie an Kieselgel mit Chloroform:Methanol=95:5 als Elutionsmittel erhält man 0,27 g (43%) Produkt. Schmelzpunkt 244 - 246 °C (Methanol).

Analog dazu wird hergestellt:
5-Phenyl-8-(trifluormethyl)-11H-1,3-dioxolo[4,5-h][1,2,4]triazolo[4,3-c][2,3]benzodiazepin

### Beispiel 24

### 5-(4-Aminophenyl)-8-(trifluormethyl)-11H-1,3-dioxolo[4,5-h][1,2 4]triazolo[4,3c][2,3]benzodiazepin

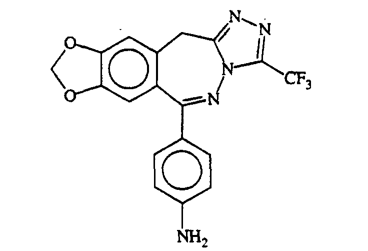

Die nach Beispiel 23 erhaltene Nitroverbindung wird analog Beispiel 2 reduziert. Ausbeute 68%, Schmelzpunkt 206 - 208 °C (Methanol).

### Beispiel 25

### 5-(4-Nitrophenyl)-11H-1,3-dioxolo[4,5-h]imidazo[1,2-c][2,3]benzodiazepin

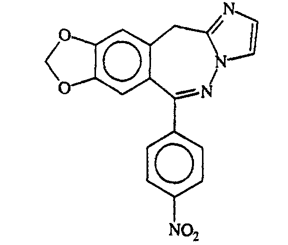

0,53 g (1,50 mmol) des in Beispiel 15E erhaltenen Methylthio-Derivates werden mit 0,10 g p-Toluolsulfonsäure und 0,32 g (3,00 mmol) Aminoacetataldehyddimethylacetal auf 120 °C erhitzt. Nach 10 Stunden wird die Reaktionsmischung in Wasser gegossen und die ausgefallene Zwischenverbindung abfiltriert. Diese Verbindung wird in 20 ml einer Mischung von konz. HCI und Ethanol 1 : 1 gelöst und 4 Stunden zum Sieden erhitzt. Nach dem Abkühlen erhält man das Hydrochlorid der Titelverbindung durch Filtration. Ausbeute 0,32g (55%), Schmelzpunkt 237 - 239 °C.

In grundsätzlich analoger Weise werden hergestellt
5-Phenyl-11H-1,3-dioxolo[4,5-h]imidazo[1,2-c][2,3]benzodiazepin

### Beispiel 26

### 5-(4-Aminophenyl)-11H-1,3-dioxolo[4,5-h]imidazo[1,2-c][2,3]benzodiazepin

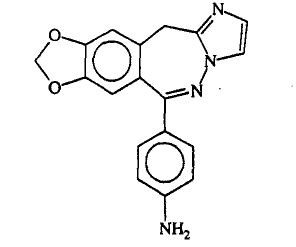

Die in Beispiel 25 hergestellte Nitroverbindung wird analog Beispiel 2 reduziert. Ausbeute 0,2 g (76%), Schmelzpunkt 264 - 265 °C (Ethanol).

### Beispiel 27

### 6-(4-Aminophenyl)-8-methoxy-3-propyl-11H-[1,2,4]triazolo[4,3-c][2,3]benzodiazepin

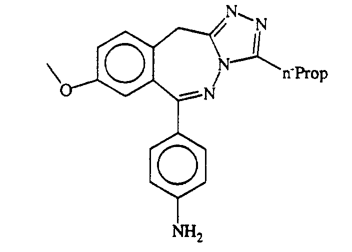

**A.**
   3,90 g (10.0 mmol) 7-Brom-8-methoxy-1-(4-nitrophenyl)-4,5-dihydro-3H-2,3-benzodiazepin-4-on werden in wasserfreiem Pyridin gelöst und mit (5,70 g (25,6 mmol) Phosphorpentasulfid versetzt. Nach 2 Stunden bei 80 °C wird das Gemisch auf 450 g Eis gegossen und 1 Stunde gerührt. Die ausgefallenen Kristalle werden filtriert und mit Wasser gewaschen. Nach Umkristallisieren aus Acetonitril erhält man 2,88 g (71%) 7-Brom-8-methoxy-1-(4-nitrophenyl)-4,5-dihydro-3H-2,3-benzodiazepin-4-thion vom Schmelzpunkt 245 - 247 °C.
**B.**
   2,84 g (7.0 mmol) der Verbindung aus Schritt A werden in 10 ml wasserfreiem DMF und 100 ml Aceton gelöst und nach Zugabe von 1,93 g (14.0 mmol) Kaliumcarbonat und 1,75 ml (28.0 mmol) Methyljodid wird das Gemisch 20 stunden bei Raumtemperatur gerührt. Das Aceton wird anschließend abgezogen und der Rückstand wird in 80 ml Wasser aufgenommen. Die ausgefallenen Kristalle werden abgesaugt und mit Wasser gewaschen. Man kristallisiert das Rohprodukt zweimal aus Acetonitril um. Man erhält 1,68 g (57%) 7-Brom-8-methoxy-4-methylthio-1-(4-nitrophenyl)-5H-2,3-benzodiazepin . vom Schmelzpunkt 225 - 227 °C.
**C.**
   1.17 g (2.78 mmol) der Verbindung aus Schritt B werden in 40 ml wasserfreiem DMF gelöst und mit 0,73 g (8.4 mmol) Buttersäurehydrazid sowie 3 Tropfen konz. HCl versetzt. Man rührt das Gemisch 5 Stunden bei 110 - 115 °C. Anschließend wird das Gemisch auf Eis (160 g) gegossen und 1 Stunde nachgerührt. Die ausgefallenen Kristalle werden abgesaugt und mit Wasser gewaschen. Man erhält 1,05 g (83%) 9-Brom-6-(4-nitrophenyl)-8-methoxy-3-propyl-11H-[1,2,4]triazolo[4,3-c][2,3]benzodiazepin vom Schmelzpunkt 238 - 240 °C.
   8-Methoxy-3-methyl-6-(4-nitrophenyl)-11H-[1,2,4)triazolo[4,3-c] [2,3]benzodiazepin
   3-Ethyl-8-methoxy-6-(4-nitrophenyl)-11H-[1,2,4]triazolo[4,3-c][2,3]benzodiazepin
   3-Cyclopropyl-8-methoxy-6-(4-nitrophenyl)-11H-[1,2,4]triazolo[4,3-c][2,3]benzodiazepin
**D.**
   1,0 g (2.2. mmol) der Verbindung aus Schritt C werden in einem Gemisch von 80 ml Methanol und 3 ml Wasser gelöst und nach Zugabe von 0.8 g 10% Pd/C Katalysator und 0.30 g (2.2 mmol) Kaliumcarbonat wird ca. 15 Stunden hydriert. Anschließend wird vom Katalysator abgesaugt und das Filtrat eingedampft. Das Rohprodukt wird aus Essigester (3 ml) umkristallisiert und man erhält 0,44 g (58%) 6-(4-Aminophenyl)-8-methoxy-3-propyl-11H-[1,2,4]triazolo[4,3-c][2,3]benzodiazepin vom Schmelzpunkt 192 .- 194 °C.

In grundsätzlich analoger Weise werden hergestellt:
6-(4-Aminophenyl)-8-methoxy-3-methyl-11H-[1,2,4]triazolo[4,3-c][2,3]benzodiazepin
6-(4-Aminophenyl)-8-methoxy-3-ethyl-11H-[1,2,4]triazolo[4,3-c] [2,3]benzodiazepin
6-(4-Aminophenyl)-8-methoxy-3-cyclopropyl-11H-[1,2,4]triazolo[4,3-c][2,3]benzodiazepin

### Beispiel 28

### 6-(4-Aminophenyl)-8-methoxy-3-methyl-11H-imidazol[1,2-c][2,3]-benzodiazepin

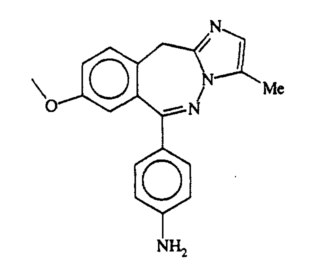

Aus 9-Brom-8-methoxy-3-methyl-6-(4-nitrophenyl)-11H-imidazo[1,2-c][2,3]benzodiazepin in Analogie zu Beispiel 27D
Schmpkt. 190-193°C (Essigester).

Grundsätzlich analog werden hergestellt:
6-(4-Aminophenyl)-8-methoxy-2-methyl-11H-imidazo[1,2-c][2,3]benzodiazepin, Schmpkt. 255-260°C (Ethanol), (Ausgangsmaterial aus Beispiel 39).
6-(4-Aminophenyl)-8-methoxy-3-n-propyl-11H-imidazo[1,2-c][2,3]benzodiazepin, Schmpkt 183-185°C, (Ausgangsmaterial aus Beispiel 41).

### Beispiel 29

### 9-Methyl-5-(4-nitrophenyl)-11H-1,3-dioxolo[4,5-h]imidazo[1,2-c][2,3]benzodiazepin

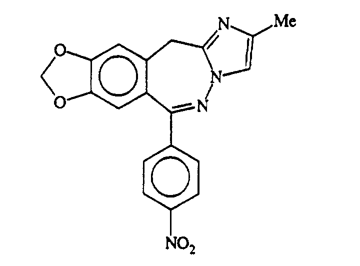

1,70 g (4,99 mmol) 5-(4-Nitrophenyl)-8,9-dihydro-7H-1,3-dioxolo[4,5-h][2,3]-benzodiazepin-8-thion (Beispiel 15D) und 1,17 g (10,0 mmol) 2-(1-Aminoethyl)-1,3-dioxolan (Shinzo Kano u. a.: Heterocycles 26, **1987**, 2805) werden mit 1,08 g rotem Quecksilberoxyd in Methylcellosolv® (50 ml) gerührt und für 36 Stunden auf 120 °C geheizt. Das Gemisch wird anschliessend filtriert und auf ein Volumen von 5 ml eingeengt. Das sich beim Abkühlen ausscheidende Zwischenprodukt wird abgesaugt und in einer Mischung von konzentrierter Salzsäure und Ethanol 1:1 (25 ml) gelöst und 1,5 Stunden zum Sieden erhitzt. Nach dem Abkühlen isoliert man das Hydrochlorid der Titelverbindung.
Ausbeute: 0,70 g (35 %), Schmelzpunkt 252-254 °C.

### Beispiel 30

### 5-(4-Aminophenyl)-9-methyl-11H-1,3-dioxolo[4,5-h]imidazo[1,2-c][2,3]benzodiazepin

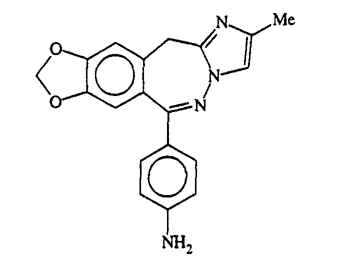

Die Reduktion der Verbindung des Beispiels 29 wird gemäß Beispiel 2 durchgeführt. Ausbeute: 0,37 g (62 %), Schmelzpunkt 165-166 °C (Ethanol).

### Beispiel 31

### 8-Cyclopropyl-5-(4-nitrophenyl)-11H-1,3-dioxolo[4,5-h]imidazo[1,2-c][2,3]benzodiazepin

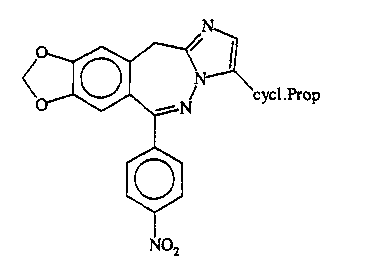

Eine Suspension von 0,50 g (1,47 mmol) 5-(4-Nitrophenyl)-8,9-dihydro-7H-1,3-dioxolo[4,5-h][2,3]benzodiazepin-8-thion (Beispiel 15D) und 0,42 g (2.94 mmol) 2-Aminomethyl-2-cyclopropyl-1,3-dioxolan in 12 ml Methylcellosolv® mit 0,32 g (1,47 mmol) rotem Quecksilberoxyd wird 12 Stunden bei 110 °C gerührt. Nach Filtration wird das Gemisch auf ein Volumen von 5 ml eingeengt und in Wasser gegossen. Der Niederschlag wird abgesaugt und in 10 ml einer 1:1 Mischung aus konzentrierter Salzsäure und Eisessig gelöst und 1,5 Stunden zum Sieden erhitzt. Beim Abkühlen scheidet sich das Hydrochlorid Salz der Titelverbindung aus.
Ausbeute: 0,35 g (56 %), Schmelzpunkt 223-225 °C.

### Beispiel 32

### 5-(4-Aminophenyl)-8-cyclopropyl-11H-1,3-dioxolo[4,5-h]imidazo[1,2-c][2,3]benzodiazepin

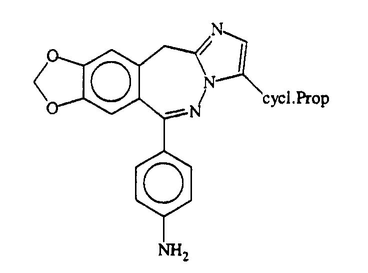

Die nach Beispiel 31 hergestellte Nitroverbindung wird analog Beispiel 2 reduziert. Das Rohprodukt wird durch Säulenchromatographie gereinigt (Kieselgel, Elutionsmittel Chloroform:Methanol=95:5).
Ausbeute: 0,25 g (85 %), Schmelzpunkt 227-229 °C (Ethanol).

### Beispiel 33

### 8-Methyl-5-(4-nitrophenyl)-11H-1,3-dioxolo[4,5-h]imidazo[1,2-c][2,3]benzodiazepin

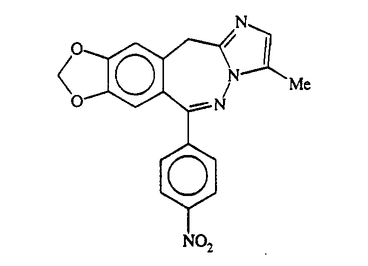

Aus 2,0 g (5,86 mmol) der Thion-Verbindung aus Beispiel 15D und 1,37g (11,71 mmol) 2-Aminomethyl-2-methyl-1,3-dioxolan (Jiro Adachi and Nobuhiro Sato: J. Org. Chem. *37*, 1972, 221) mit 1,27 g (5,86 mmol) rotem Quecksilberoxyd wird analog zum Beispiel 31 gearbeitet. Die isolierte Zwischenverbindung wird durch ein 5-stündiges Kochen in 50 ml Eisessig zum Ringschluß gebracht. Anschließend wird zur Trockene eingeengt und der Rückstand wird in 10%iger Natriumcarbonat-Lösung und Essigester aufgelöst. Nach dem Einengen der organischen Phase wird das Rohprodukt säulenchromatographisch gereinigt (Kieselgel, Elutionsmittel Chloroform:Methanol=95:5).
Ausbeute: 0,80 g (38 %), Schmelzpunkt 220-222 °C.

### Beispiel 34

### 5-(4-Aminophenyl)-8-methyl-11H-1,3-dioxolo[4,5-h]imidazo[1,2-c][2,3]benzodiazepin

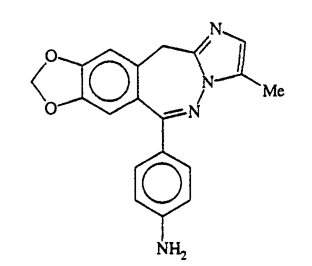

Die nach Beispiel 34 hergestellte Nitroverbindung wird analog Beispiel 2 reduziert.
Ausbeute: 0,51 g (68 %), Schmelzpunkt 283-285 °C (Ethanol).

### Beispiel 35

### 8-Methyl-5-(4-nitrophenyl)-11H-1,3-dioxolo[4,5-h]imidazo[3,4-c][2,3]benzodiazepin

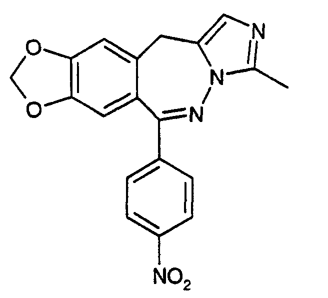

### A. 5-(4-Nitrophenyl)-8-(phtalimidomethyl)-9H-1,3-dioxolo[4,5-h][2,3]-benzodiazepin

Zu einer gerührten Lösung von 2,60g (7,66 mmol) 8-Hydroxymethyl-5-(4-nitrophenyl)-9H-1,3-dioxolo[4,5-h][2,3]-benzodiazepin (Ausgangsverbindung III.), 2,11 g (8,05 mmol) Triphenylphosphin und 1,18g (8,05 mmol) Phtalimid in 130 ml trockenem THF wird bei Raumtemperatur eine Lösung von 1,25 ml (8,05 mmol) Diethylazodicarboxylat in 7 ml THF getropft. Das Gemisch wird 24 Stunden bei dieser Temperatur gerührt. Dann wird das ausgeschiedene Produkt abgesaugt und mit Ethanol gewaschen. Man erhält 2,87 g (80 %) der Titelverbindung, die ohne weitere Reinigung weiterverarbeitet werden kann.

### B. 8-Aminomethyl-5-(4-nitrophenyl)-9H-1,3-dioxolo[4,5-h][2,3]-benzodiazepin

Zu einer Suspension von 1,10 g (2,35 mmol) der Phtalimido-Verbindung aus dem Reaktionsschritt A in 75 ml Methanol werden 0,60 ml (11,7 mmol) 98%iges Hydrazinhydrat gegeben und das Gemisch wird 3h zum Sieden erhitzt. Nach Eindampfen wird der Rückstand mit 30 ml Methylenchlorid verrieben und abfiltriert. Das Filtrat wird im Vakuum eingeengt und der Rückstand mit Wasser zum Kristallisieren gebracht. Nach Absaugen erhält man 0,72 g (90 %) des Produktes vom Schmelzpunkt 143-146 °C, das ohne weitere Reinigung für den nächsten Schritt geeignet ist.

### C. 8-Acetaminomethyl-5-(4-nitrophenyl)-9H-1,3-dioxolo[4,5-h][2,3]-benzodiazepin

0,72 g (2,13 mmol) Aminomethyl-Verbindung aus Schritt B werden in 6 ml Essigsäurenahydrid bei 25 °C gelöst und 1 Stunde stehen gelassen. Die Lösung wird mit Eiswasser (30 ml) verdünnt und 2h gerührt. Die ausgeschiedene Substanz wird filtriert und nach dem Trocknen durch Säulenchromatographie gereinigt (Kieselgel, Elutionsmittel Essigester:Benzol=4:1). Nach Eindampfen der Fraktionen werden 0,65 g kristalline Substanz erhalten, die nach Waschen mit Ethanol 0,56 g (70 %) reine Titelverbindung vom Schmelzpunkt 205 °C. (Zers.) ergeben.

### D. 8-Methyl-5-(4-nitrophenyl)-11H-1,3-dioxolo[4,5-h]imidazo[3,4-c][2,3]benzodiazepin

0,40 g (1,05 mmol) Acetamido-Verbindung aus Schritt C werden in 20 ml Methylenchlorid suspendiert und mit 0,48ml (5,3 mmol) Phosphoroxychlorid versetzt. Anschließend wird das Gemisch 3h zum Sieden erhitzt. Nach Einengen wird der Rückstand in Methylenchlorid (30 ml) aufgenommen und mit Natriumbicarbonat Lösung und Wasser gewaschen, getrocknet, filtriert und eingeengt. Man erhält 0,38 g feste Substanz, die durch Säulenchromatographie gereinigt wird (Kieselgel, Elutionsmittel Chloroform:Methanol=95:5). Es werden 0,32 g (84 %) reine Titelverbindung vom Schmelzpunkt 305-310 °C (Zers.) erhalten.

In analoger Weise werden über die entsprechenden Stufen C-D hergestellt, wobei die für die Acylierung verwendete Methode in Klammern nachgestellt ist:
5-(4-Nitrophenyl)-11H-1,3-dioxolo[4,5-h]imidazo[3,4-c] [2,3]benzodiazepin (Ameisensäure, DCC)
5-Phenyl-11H-1,3-dioxolo[4,5-h]imidazo[3,4-c][2,3]benzodiazepin (Ameisensäure, DCC)
8-Cyclopropyl-5-(4-nitrophenyl)-11H-1,3-dioxolo[4,5-h]imidazo[3,4-c][2,3]benzodiazepin (Säurechlorid)
5-(4-Nitrophenyl)-8-n-propyl-11H-1,3-dioxolo[4,5-h]imidazo[3,4-c][2,3]benzodiazepin (Säurechlorid)

### Beispiel 36

### 5-(4-Aminophenyl)-8-methyl-11H-1,3-dioxolo[4,5-h]imidazo[3,4-c][2,3]benzodiazepin

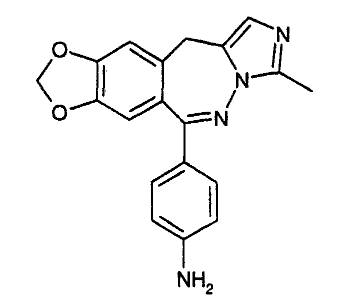

Die nach Beispiel 35 hergestellte Nitroverbindung (0,30 g, 0,83 mmol) wird analog Beispiel 2 reduziert. Man erhält 0,22 g (81 %) Titelverbindung vom Schmelzpunkt 282-284 °C (Zers.).

In analoger Weise werden hergestellt:

### 5-(4-Aminophenyl)-11H-1,3-dioxolo[4,5-h]imidazo[3,4-c][2,3]benzodiazepin

### 8-Cyclopropyl-5-(4-aminophenyl)-11H-1,3-dioxolo[4,5-h]imidazo[3,4-c][2,3]benzodiazepin

### 5-(4-Aminophenyl)-8-n-propyl-11H-1,3-dioxolo[4,5-h]imidazo[3,4-c][2,3]benzodiazepin

### Beispiel 37

### 8-Ethyl-5-(4-nitrophenyl)-11H-1,3-dioxolo[4,5-h]imidazo[3,4-c][2,3]-benzodiazepin

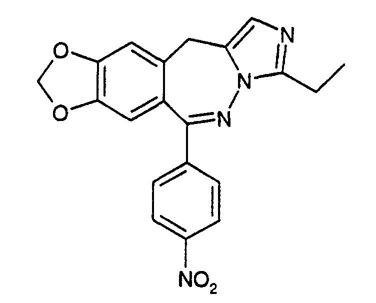

### A. 8-Azidomethyl-5-(4-nitrophenyl)-9H-1,3-dioxolo[4,5-h][2,3]-benzodiazepin

Eine Lösung von 3,06 g (9,0 mmol) 8-Hydroxymethyl-5-(4-nitrophenyl)-9H-1,3-dioxolo[4,5-h][2,3]-benzodiazepin (Ausgangsverbindung III.) und 2,58 g (9,9 mmol) Triphenylphosphin in 100 ml trockenem Tetrahydrofuran wird mit 13,5 ml einer 1,2N Stickstoffwasserstoffsäure-Lösung in Toluol versetzt, anschliessend wird eine Lösung von 1,74 ml (9,9 mmol) Azodicarbonsäure-diethylester zugegeben und das Gemisch weitere 2h gerührt. Das ausgeschiedene Produkt wird abgesaugt und mit Tetrahydrofuran und n-Hexan gewaschen. Man erhält 2,23 g (68%) der Titelverbindung vom Schmp. 198-200'C.

### B. 8-Ethyl-5-(4-nitrophenyl)-11H-1,3-dioxolo[4,5-h]imidazo[3,4-c][2,3]benzodiazepin

1,98g (5,4 mmol) der Verbindung aus dem Schritt A werden in 100 ml trockenem THF gelöst und mit 1,56 g (5,94 mmol) Triphenylphosphin versetzt und 4h gerührt. Anschliessend wird die Lösung auf -50'C gekühlt und eine Lösung von 0,78 ml (6,0 mmol) Propionsäureanhydrid in 3 ml THF wird zugegeben. Nach 1h bei -50'C wird das Gemisch über Nacht bei RT gerührt. Das Reaktionsgemisch wird danach mit Diethylether verdünnt und mit 10%iger Natriumbicarbonat-Lösung und Wasser gewaschen, getrocknet und eingedampft. Der Rückstand wird durch Säulenchromatographie an Kieselgel gereinigt. (Gradient-Elution: beginnend mit n Hexan:Essigester= 1:1, dann mit ständig wachsendem Essigester Anteil).

Man erhält 1,0g eines Produktes, das nach dem Aufkochen in 5ml Essigester 0,75g eines Substanzgemisches ergibt, das aus der Titelverbindung und der Zwischenverbindung 8-Propionylaminomethyl-5-(4-nitrophenyl)-9H-1,3-dioxolo[4,5-h][2,3]-benzodiazepin besteht.
Um den Ringschluss zu vervollständigen wird das obige Gemisch in wasserfreiem Dichlorethan gelöst und nach Zugabe von 0,20 ml (2,15 mmol) Phosphoroxychlorid 2h zum Sieden erhitzt. Das abgekühlte Reaktionsgemisch wird anschliessend mit Natriumbicarbonat-Lösung gewaschen und zur Trockene eingedampft.
Man erhält 0,65 g (33%) der Titelverbindung vom Schmp. 243-245°C.

### Beispiel 38

### 5-(4-Aminophenyl)-8-ethyl-11H-1,3-dioxolo[4,5-h]imidazo[3,4-c][2,3]benzodiazepin

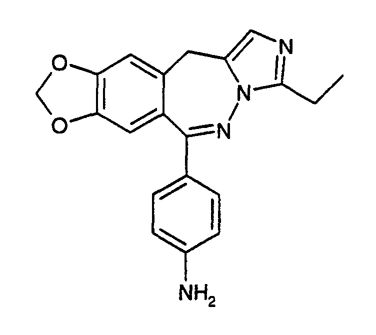

0,38 g (1,0 mmol) der nach dem Beispiel 37 hergestellten Nitroverbindung wird in 10 ml eines 1:1 Gemisches aus Methylenchlorid und Methanol nach Beispiel 2 reduziert. Das Rohprodukt wird durch Säulenchromatographie (Kieselgel, Elutionsmittel:
Chloroform:Methanol=95:5) gereinigt.
Man erhält 0,28 g (81 %) der Titelverbindung vom Schmp. 135-138°C.

### Beispiel 39

### 9-Ethyl-5-(4-nitrophenyl)-11H-1,3-dioxolo[4,5-h]imidazo59[1,2-c][2,3]benzodiazepin

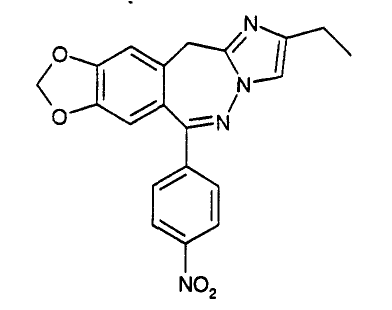

Ein Gemisch aus 1,0 g (2,82 mmol) 8-Methylthio-5-(4-nitrophenyl)-9H-1,3dioxolo[4,5-h][2,3]-benzodiazepin (Beispiel 15, Schritt E) und 0,74 g (5,64 mmol) 2-(1-aminopropyl)-1,3-dioxolan (J. Org. Chem. 21, **1956**, 115) in 60 ml Methylcellosolve® wird nach der Zugabe von einer katalytischen Menge p-Toluolsulfonsäure 48h bei 120°C gerührt. Nach dem Abkühlen wird von der unveränderten Methylthio-Verbindung abfiltriert und das Filtrat auf ein Volumen von 10ml eingeengt. Nach der Zugabe von 50ml Wasser fällt die Zwischenverbindung des Kondensationsschrittes aus und wird abgesaugt. Der Filterrückstand wird in 10 ml Ethanol:konzentrierter Salzsäure=1:1 gelöst und 1,5h zum Sieden erhitzt. Anschliessend wird die Lösung eingedampft und der Rückstand in 50ml Wasser aufgenommen. Es wird mit Natriumcarbonat neutralisiert und mit Essigester extrahiert, getrocknet, filtriert und eingeengt. Der Rückstand wird durch Säulenchromatographie (Kieselgel, Elutionsmittel: Chloroform:Methanol=95:5) gereinigt. Man erhält 0,38 g (36%) der Titelverbindung vom Schmp. 188-190'C.

In analoger Weise werden hergestellt:
9-Brom-8-methoxy-3-methyl-6-(4-nitrophenyl)-11H-imidazo[1,2-c][2,3]benzodiazepin, Schmpkt: 196-200°C
9-Brom-8-methoxy-2-methyl-6-(4-nitrophenyl)-11H-imidazo[1,2-c] [2,3]benzodiazepin, Schmpkt. 265-268°C (Ethanol).
(Ausgangsmaterial ist jeweils die Verbindung aus Beispiel 27 B)

### Beispiel 40

### 5-(4-Aminophenyl)-9-ethyl-11H-1,3-dioxolo[4,5-h]imidazo[1,2-c][2,3]benzodiazepin

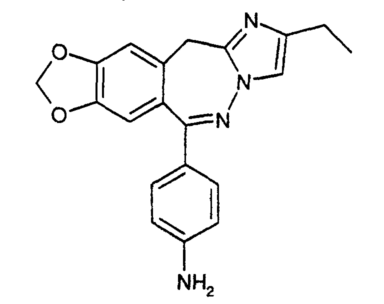

Die Nitroverbindung des Beispiels 39 wird analog Beispiel 2 in Methylenchlorid:Methanol=1:1 reduziert. Nach dem Umkristallisieren aus Essigester erhält man 0,14g (41%) der Titelverbindung vom Schmp. 192-194°C.

### Beispiel 41

### 5-(4-Nitrophenyl)-8-propyl-11H-1,3-dioxolo[4,5-h]imidazo[1,2-c][2,3]benzodiazepin-hydrochlorid

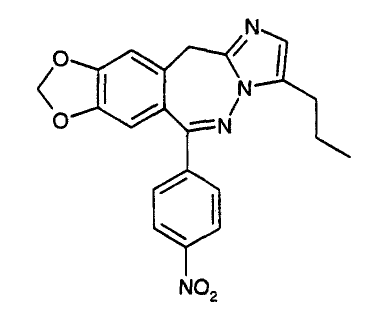

Hergestellt aus 0,68g (2,0 mmol) der Thion-Verbindung (Beispiel 15, Schrift D) und 0,58g (4,0 mmol) 2-Aminomethyl-2-propyl-1,3-dioxolan (hergestellt analog J. Org. Chem., 37, **1972**, 221) und 0,43g (2,0 mmol) rotem Qecksilberoxyd nach Beispiel 29. Nach 10h bei 110°C wird die Zwischenverbindung des Kondensationsschrittes chromatographisch gereinigt (Kieselgel, Elutionsmittel: Chloroform-Methanol=95:5). Ringschlussreaktion erfolgt durch Erhitzen des Zwischenproduktes in einem 1:1 Gemisch aus Essigsäure und konzentrierter Salzsäure.
Nach dem Eindampfen erhält man 0,36g der die Titelverbindung als Hydrochlorid-Salz. Ausbeute 42%, Schmp. 200-201°C.

Analog wird hergestellt:

### 9-Brom-8-methoxy-6-(4-nitrophenyl)-3-n-propyl-11H-imidazo[1,2-c][2,3]benzodiazepin, Schmpkt 150-162°C

### Beispiel 42

### 5-(4-Aminophenyl)-8-propyl-11H-1,3-dioxolo[4,5-h]imidazo[1,2-c][2,3]benzodiazepin

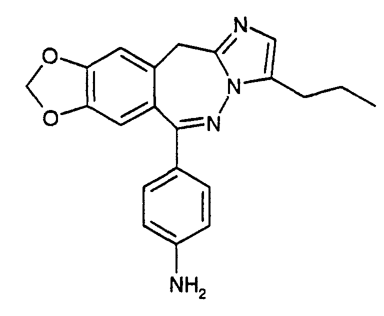

Die Nitroverbindung des Beispiels 41 wird analog Beispiel 2 reduziert. Nach Umkristallisieren aus Ethanol erhält man 0,27g (89%) der Titelverbindung vom Schmp. 175-176°C (aus Ethanol).

### Beispiel 43

### 8-Ethyl-5-(4-nitrophenyl)-11H-1,3-dioxolo[4,5-h]imidazo(1,2-c][2,3]-benzodiazepinhydrochlorid

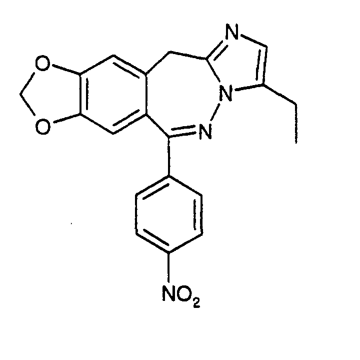

Hergestellt analog Beispiel 41 aus 0,68g (2,0 mmol) der Thion-Verbindung (Beispiel 15, Schritt D) und 0,53g (4,0 mmol) 2-Aminomethyl-2-ethyl-1,3dioxolan (Herstellung analog J. Org. Chem., 37, **1972**, 221). Die Titelverbindung wird als Hydrochlorid (0,32g) isoliert. Ausbeute: 39%, Schmp. 217-218°C.

### Beispiel 44

### 5-(4-Aminophenyl)-8-ethyl-11H-1,3-dioxolo[4,5-h]imidazo[1,2-c][2,3]benzodiazepin

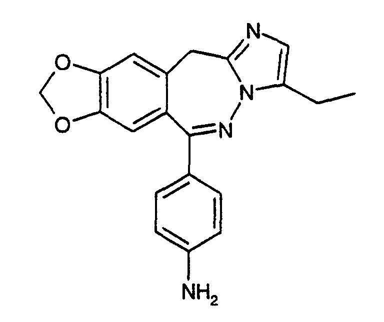

Hergestellt aus der Nitroverbindung des Beispiels 43 nach Beispiel 2. Man erhält 0,18g der Titelverbindung. Ausbeute: 67%,Schmp. 258-260°C (Ethanol).

### Beispiel 45

### 8,9-Dimethyl-5-(4-nitrophenyl)-11H-1,3-dioxolo[4,5-h]imidazo[1,2-c][2,3]benzodiazepin hydrochlorid

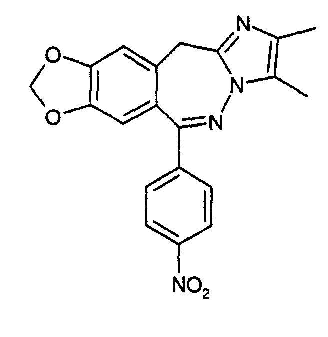

Die Titelverbindung wird analog Beispiel 29 aus 2,0g (5,86 mmol) der Thiooxo-Verbindung des Beispiels 15, Schritt D und 1,54g (11,72 mmol) 2-(1-aminoethyl)-2-methyl-1,3-dioxolan (J. Org. Chem. 37, **1972**, 221) mit 1,279 (5,86 mmol) rotem Qecksilberoxyd hergestellt. Der Kondensationsschritt dauert bei 110°C ca. 30h. Das Zwischenprodukt wird wie beim Beispiel 41 chromatographisch gereingt und anschliessend erfolgt der Ringschluss durch Kochen in 10m eines Gemisches aus Ethanol-konzentrierter Salzsäure. Die Titelverbindung wird als Hydrochlorid isoliert: 0,52g (22%), Schmp. 240-243°C.

Analog wird hergestellt:
9-Brom-2,3-dimethyl-8-methoxy-6-(4-nitrophenyl)11H-imidazo[1,2-c][2,3]benzodiazepin, Schmpkt: 190-193°C

### Beispiel 46

### 5-(4-Aminophenyl)-8,9-dimethyl-11H-1,3-dioxolo[4,5-h]imidazo[1,2-c]-[2,3]benzodiazepin

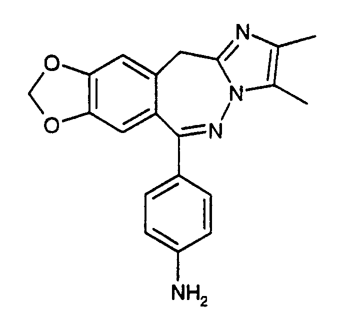

Die Nitroverbindung des Beispiels 45 wird gemäss Beispiel 2 reduziert. Man erhält 0.33g (75%) der Titelverbindung vom Schmp. 226-227°C (Ethanol).

### Beispiel 47

### A. 4-Nitrobenzoylhydrazons vom 2-Hydroxy-4-methoxybenzaldehyd

63,4g 4-Nitrobenzhydrazid werden in 2,51 1-Propanol vorgelegt und mit 53,3g 2-Hydroxy-4-methoxybenzaldehyd versetzt und 1h am Rückfluss gekocht. Nach Kühlen im Eisbad wird abgesaugt. Man erhält 104g des 4-Nitrobenzoylhydrazons vom 2-Hydroxy-4-methoxybenzaldehyd.

Analog werden hergestellt:
Benzoylhydrazon vom 2-Hydroxy-4-methoxybenzaldehyd
4-Brombenzoylhydrazon vom 2-Hydroxy-4-methoxybenzaldehyd

### B. 2-(4-Nitrobenzoyl)-4-methoxybenzaldehyd

50g 4-Nitrobenzoylhydrazon vom 2-Hydroxy-4-methoxybenzaldehyd werden in 1,51 Tetrahydrofuran (getrocknet über Molsieb) bei 8°C vorgelegt und portionsweise mit 99,4g Blei(IV)acetat (85%ig) versetzt. Nach vollendeter Zugabe wird 30min nachgerührt, abgesaugt und das Filtrat eingeengt. Der Rückstand wird in Essigester aufgenommen, nacheinander mit Wasser und Kochsalzlösung gewaschen, getrocknet, filtriert und eingeengt. Nach Umkristallisation aus Essigester erhält man 22,8g 2-(4-Nitrobenzoyl)-4-methoxybenzaldehyd.

In analoger Weise werden hergestellt:
2-Benzoyl-4-methoxybenzaldehyd
2-(4-Brombenzoyl)-4-methoxybenzaldehyd

### C. 1-Methoxy-2-(4-methoxy-2-(4-nitrobenzoyl)phenyl)ethylen.

10g 2-(4-Nitrobenzoyl)-4-methoxybenzaldehyd werden zusammen mit 18g Methoxymethyltriphenylphosphoniumchlorid in 400ml Toluol vorgelegt und unter Eiskühlung portionsweise mit 5,9g Kalium-tert.-butylat versetzt. Nach 1h Rühren bei Eiskühlung und anschliessendem 3,5stündigem Rühren bei Raumtemperatur wird mit 200ml Wasser versetzt, mit IN-Salzsäure schwach angesäuert und dreimal mit Essigester extrahiert. Die Essigesterphasen werden mit gesättigter Kochsalzlösung gewaschen, getrocknet filtriert und eingeengt. Der Rückstand wird über Kieselgel mit Hexan:Essigester=1:1 als Elutionsmittel chromatographiert. Man erhält 6,9g 1-Methoxy-2-(4-methoxy-2-(4-nitrobenzoyl)phenyl)ethylen als Gemisch der E- und der Z-Form.

In analoger Weise werden hergestellt:
1-Methoxy-2-(4-methoxy-2-benzoylphenyl)ethylen.
1-Methoxy-2-(4-methoxy-2-(4-brombenzoyl)phenyl)ethylen.

### D. 2-(4-Methoxy-2-(4-nitrobenzoyl)phenyl)essigsäure

6,9g 1-Methoxy-2-(4-methoxy-2-(4-nitrobenzoyl)ethylen als Gemisch der E- und der Z-Form werden in 310ml Tetrahydrofuran vorgelegt und mit 100ml 1-N Salzsäure versetzt. Nach Rühren über Nacht bei Raumtemperatur wird mit 300ml Wasser verdünnt und das Tetrahydrofuran bei 30°C Badtemperatur abdestilliert. Die wässrige Phase wird dreimal mit Essigester extrahiert. Die gesammelte organische Phase wird mit Wasser gewaschen, getrocknet, filtriert und eingeengt. Es wird in 300ml Aceton aufgenommen und bei 4°C tropfenweise mit 11,8ml 8N-Jones-Reagenz versetzt. Nach beendeter Zugabe wird 2h bei dieser Temperatur nachgerührt, mit 6ml Isopropanol versetzt und weitere 15min nachgerührt. Es wird dann mit 200ml Wasser verdünnt und das Aceton am Rotationsverdampfer abgezogen. Die wässrige Phase wird dreimal mit Essigester extrahiert und die gesammelte organische Phase mit Wasser gewaschen, getrocknet, filtriert und eingeengt. Nach Umkristallisation aus Essigester/Hexan erhält man 6,3g 2-(4-Methoxy-2-(4-nitrobenzoyl)phenyl)essigsäure.

In analoger Weise werden hergestellt:
2-(4-Methoxy-2-benzoylphenyl)essigsäure
2-(4-Methoxy-2-(4-brombenzoyl)phenyl)essigsäure

### E. 8-Methoxy-1-(4-nitrophenyl)-4,5-dihydro-3H-2,3-benzodiazepin-4-on

7,8g 2-(4-Methoxy-2-(4-nitrobenzoyl)phenyl)essigsäure werden in 200ml Tetrahydrofuran mit 2,3ml 80%igem Hydrazinhydrat versetzt und für 6h bei Raumtemperatur gerührt. Nach Stehen über Nacht wird mit 50ml Wasser versetzt und das Tetrahydrofuran am Rotationsverdampfer abgezogen. Das ausgefallene 2-(4-Methoxy-2-(4-nitrobenzoyl)phenyl)essigsäurehydrazid (4,9g) wird abgesaugt und in 37ml Eisessig bei Raumtemperatur 2h gerührt. Es wird mit 37ml Wasser verdünnt und abgesaugt. Man erhält 4,37g 8-Methoxy-1-(4-nitrophenyl)-4,5-dihydro-3H-2,3-benzodiazepin-4-on vom Schmelzpkt 282°C.

In grundsätzlich analoger Weise, aber über das gemischte Säureanhydrid mit Chlorameisensäureisobutylester werden hergestellt:
8-Methoxy-1-phenyl-4,5-dihydro-3H-2,3-benzodiazepin-4-on
8-Methoxy-1-(4-bromphenyl)-4,5-dihydro-3H-2,3-benzodiazepin-4-on

### F. 8-Methoxy-1-(4-nitrophenyl)-4,5-dihydro-3H-2,3-benzodiazepin-4-thion

4,3g 8-Methoxy-1-(4-nitrophenyl)-4,5-dihydro-3H-2,3-benzodiazepin-4-on werden in 48ml Pyridin mit 2,46g Diphosphorpentasulfid versetzt und unter Argon und Feuchtigkeitsausschluss für 2h bei 100°C Badtemperatur gerührt. Es wird mit Wasser verdünnt und das ausgefallene Produkt abgesaugt. Nach Chromatographie über Kieselgel zunächst mit Essigester:Hexan=1:1 und später mit Essigester erhält man insgesamt 3,13g 8-Methoxy-1-(4-nitrophenyl)-4,5-dihydro-3H-2,3-benzodiazepin-4-thion.

In analoger Weise werden hergestellt:
8-Methoxy-1-phenyl-4,5-dihydro-3H-2,3-benzodiazepin-4-thion
8-Methoxy-1-(4-bromphenyl)-4,5-dihydro-3H-2,3-benzodiazepin-4-thion

### G. 8-Methoxy-3-methyl-6-phenyl-11H-imidazo[1,2-c][2,3]benzodiazepin

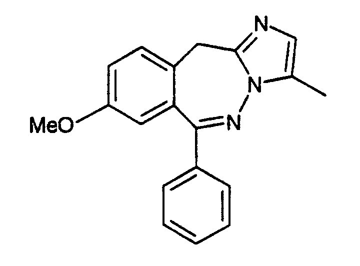

500mg 8-Methoxy-1-(-phenyl)-4,5-dihydro-3H-2,3-benzodiazepin-4-thion werden in 1,5ml Ethylenglykolmonomethylether (Cellosolve®) und 548mg 2-Aminomethyl-2-methyl-1,3-dioxolan unter Durchleitung von Argon 10h bei 60°C gerührt. Nach Filtration und Waschen mit kaltem Ethanol und Diisopropylether erhält man 550mg Iminoverbindung, die in 10ml Ethanol gelöst, mit 10ml konzentrierter Salzsäure versetzt und 3h am Rückfluss gekocht wird. Es wird in Wasser gegeben, auf pH11 gestelltund mit Essigester extrahiert. Die Essigesterphase wird mit Wasser gewaschen, getrocknet, filtriert und eingeengt. Nach Umkristallisation aus Essigester/ Diisopropylether erhält man 240mg 8-Methoxy-3-methyl-6-phenyl-11H-imidazo[1,2-c] [2,3]benzodiazepin vom Schmelzpunkt 140°C.

In analoger Weise werden aus den entsprechenden Thionen hergestellt:
8-Methoxy-2-methyl-6-phenyl-11 H-imidazo[1,2-c] [2,3]benzodiazepin
8-Methoxy-3-methyl-6-phenyl-11H-imidazo[1,2-c][2,3]benzodiazepin
8-Methoxy-3-ethyl-2-methyl-6-phenyl-11H-imidazo[1,2-c] [2,3]benzodiazepin
8-Methoxy-6-phenyl-3-(4-pyridyl)-11H-imidazo[1,2-c][2,3]benzodiazepin²
8-Methoxy-6-phenyl-3-(2-pyridyl)-11H-imidazo[1,2-c][2,3]benzodiazepin²
8-Methoxy-6-phenyl-3-(3-pyridyl)-11H-imidazo[1,2-c][2,3]benzodiazepin²
3,6-Diphenyl-8-methoxy-2-methyl-11H-imidazo[1,2-c] [2,3]benzodiazepin
8-Methoxy-6-(4-nitrophenyl)-3-(2-pyridyl)-11H-imidazo[1,2-c] [2,3]benzodiazepin
8-Methoxy-6-(4-nitrophenyl)-3-(4-pyridyl)-11H-imidazo[1,2-c] [2,3]benzodiazepin
5-(4-Chlorphenyl)-8,9-dimethyl-11H-1,3-dioxolo[4,5-h]imidazo[1,2-c][2,3]benzodiazepin
9-Ethyl-8-methyl-5-(4-nitrophenyl)-11H-1,3-dioxolo[4,5-h]imidazo[1,2-c] [2,3]benzodiazepin
8-Ethyl-9-methyl-5-(4-nitrophenyl)-11H-1,3-dioxolo[4,5-h]imidazo[1,2-c] [2,3]benzodiazepin
5-(4-Nitrophenyl)-8-(4-pyridyl)-11H-1,3-dioxolo[4,5-h]imidazo[1,2-c][2,3]benzodiazepin²
8,9-Dimethyl-5-phenyl-11H-1,3-dioxolo[4,5-h]imidazo[1,2-c][2,3]benzodiazepin

² Die entsprechenden Ketale werden nach Org.Synth. **64**, 19,(1986) hergestellt

### Beispiel 48

### 2,3-Dimethyl-6-(4-nitrophenyl)-8-methoxy-11H-imidazo[1,2-c][2,3]benzodiazepin

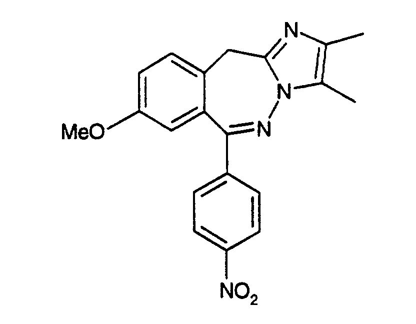

2,3g 8-Methoxy-1-(4-nitrophenyl)-4,5-dihydro-3H-2,3-benzodiazepin-4-thion werden mit 3ml 2-Amino-3,3-dimethoxybutan (hergestellt durch reduktive Aminierung nach J.Org.Chem. 52,(12),2616 aus 3,3-Dimethoxybutan-2-on) unter Durchleiten von Argon 4h bei 110°C Badtemperatur gerührt. Der Ansatz wird mit 50ml 1N-Salzsäure versetzt, mit Wasser auf 100ml verdünnt und dreimal mit je 150ml Essigester extrahiert. Die wässrige Phase wird mit 1-N Natronlauge alkalisch gestellt und dreimal mit Essigester extrahiert. Die gesammelten organischen Phasen werden getrocknet, filtriert und eingeengt und der Rückstand über Kieselgel mit Methylenchlorid:Etanol=10:1 als Elutionsmittel chromatographiert.Man erhält 1,5g 2,3-Dimethyl-8-methoxy-6-(4-nitrophenyl)-11H-imidazo[1,2-c][2,3]benzodiazepin.

In analoger Weise werden hergestellt:
6-(4-Bromphenyl)-2,3-dimethyl-8-methoxy-11H-imidazo[1,2-c][2,3]benzodiazepin
2,3-Dimethyl-8-methoxy-6-phenyl-11H-imidazo[1,2-c] [2,3]benzodiazepin
8,9-Dimethyl-5-(4-fluorphenyl)-11H-1,3-dioxolo[4,5-h]imidazo[1,2-c][2,3]benzodiazepin

### Beispiel 49

### A. 6-(4-Aminophenyl)-2,3-dimethyl-8-methoxy-11H-imidazo[1,2-c][2,3]benzodiazepin

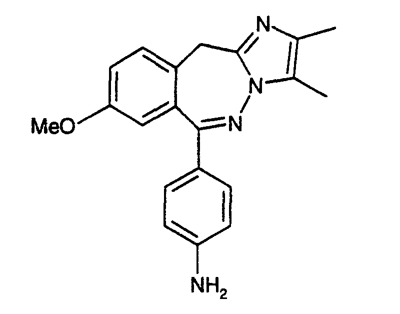

834mg 2,3-Dimethyl-8-methoxy-6-(4-nitrophenyl)-11H-imidazo[1,2-c] [2,3]benzodiazepin in 33ml Eisessig zusammen mit 2,25g Eisenpulver in einem auf 90°C vorgeheizten Ölbad 20min. erhitzt. Es wird heiss abgesaugt und mit Eisessig nachgewaschen. Das Filtrat wird eingeengt und der Rückstand in Essigester und 1N-Natronlauge verteilt. Die wässrige Phase wird noch zweimal mit Essigester ausgeschüttelt und die gesammlte organische Phase wird mit Wasser gewaschen, getrocknet, filtriert und eingeengt. Dieser Rückstand wird über Kieselgel mit Methylenchlorid:Ethanol=10:1 als Elutionsmittel chromatographiert. Nach Ausrühren der entsprechenden eingeengten Fraktionen mit Essigester/Hexan erhält man 331mg 6-(4-Aminophenyl)-2,3-dimethyl-8-methoxy-11H-imidazo[1,2-c][2,3]benzodiazepin vom Schmelzpunkt 280°C.

In analoger Weise werden hergestellt:
6-(4-Aminophenyl)-8-methoxy-3-(2-pyridyl)- 11H-imidazo[1,2-c][2,3]benzodiazepin
6-(4-Aminophenyl)-8-methoxy-3-(4-pyridyl)-11H-imidazo[1,2-c][2,3]benzodiazepin
5-(4-Aminophenyl)-9-brom-8-methyl-11H-1,3-dioxolo[4,5-h]imidazo[1,2-c] [2,3]benzodiazepin
5-(4-Aminophenyl)-8-brom-9-methyl-11H-1,3-dioxolo[4,5-h]imidazo[1,2-c] [2,3]benzodiazepin

### B. 5-(4-Aminophenyl)-8-ethyl-9-methyl-11H-1,3-dioxolo[4,5-h]imidazo[1,2-c][2,3]benzodiazepin

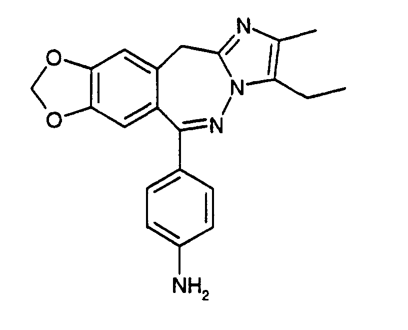

527mg 8-Ethyl-9-methyl-5-(4-nitrophenyl)-11H-1,3-dioxolo[4,5-h]imidazo[1,2-c][2,3]benzodiazepin werden in 11ml Ethanol mit 5,4ml Cyclohexen und 106mg Palladiumhydroxid auf Kohle (Pearlmans Kat.) versetzt und 3h bei 110°C Badtempereatur gerührt. Nach Abfiltrieren vom Katalysator wird eingeengt und der Rückstand über Kieselgel mit Essigester als Elutionsmittel chromatographiert. Nach Zusammenfassen der entsprechenden Fraktionen und Umkristallisation aus Ethanol erhält man 348mg 5-(4-Aminophenyl)-8-ethyl-9-methyl-11H-1,3-dioxolo[4,5-h]imidazo[1,2-c][2,3]benzodiazepin vom Schmelzpunkt 227-228°C.

In analoger Weise werden hergestellt:
5-(4-Aminophenyl)-8-(4-pyridyl)-11H-1,3-dioxolo[4,5-h]imidazo[1,2-c][2,3]benzodiazepin
5-(4-Aminophenyl)-9-ethyl-8-methyl-11H-1,3-dioxolo[4,5-h]imidazo[1,2-c] [2,3]benzodiazepin

### Beispiel 50

### 8-Methyl-5-phenyl-11H-1,3-dioxolo[4,5-h]imidazo[1,2-c][2,3]benzodiazepin

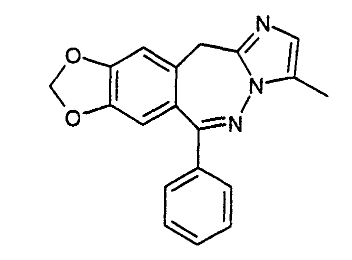

200mg 5-(4-Aminophenyl)-8-methyl-11H-1,3-dioxolo[4,5-h]imidazo[1,2-c][2,3]benzodiazepin werden in 30ml Tetrahydrofuran mit 1,32ml Pentylnitrit versetzt und unter Argon 2 h zum Rückfluss erhitzt. Nach Einengen wird über Kieselgel mit Methylenchlorid:Ethanol=10:1 chromatographiert. Man erhält 136mg 8-Methyl-5-phenyl-11H-1,3-dioxolo[4,5-h]imidazo[1,2-c][2,3]benzodiazepin.

### Beispiel 51

### 5-(4-Chlorphenyl)-8-methyl-11H-1,3-dioxolo[4,5-h]imidazo[1,2-c][2,3]benzodiazenin

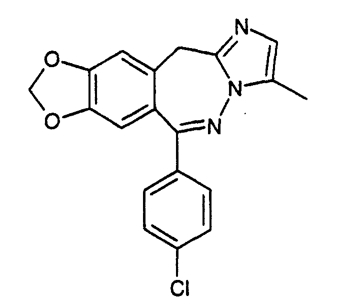

160mg 5-(4-Aminophenyl)-8-methyl-11H-1,3-dioxolo[4,5-h]imidazo[1,2-c][2,3]benzodiazepin werden 2ml Wasser und 2ml konz. Salzsäure gelöst, und bei 0°C mit einer Lösung von 36mg Natriumnitrit in 0,5ml Wasser diazotiert. Es wird 45min. bei 0°C weitergerührt. Zu dieser Lösung wird bei Raumtemperatur eine Lösung getropft, die folgendermassen bereitet wird: 256mg Kupfersulfatpentahydrat werden in 1ml Wasser mit 87mg Natriumchlorid versetzt und tropfenweise mit einer Lösung aus 68mg Natriumsulfit in 0,6ml Wasser versetzt. Der weisse Niederschlag wird vom Überstand durch Abdekantieren getrennt, zweimal mit Wasser gewaschen und in konz. Salzsäure gelöst. Nach Zugabe dieser Lösung wird 10min auf dem Dampfbad erwärmt und 1h bei Raumtemperatur nachgerührt. Es wird mit Wasser verdünnt, mit Ammoniaklösung alkalisch gestellt und mit Essigester extrahiert. Die Essigesterphase wird mit gesättigter Natriumchloridlösung gewaschen, getrocknet, filtriert und eingeengt. Nach Chromatographie über Kieselgel mit Methylenchlorid:Ethanol=10:1 erhält man 87mg 5-(4-Chlorphenyl)-8-methyl- 11H-1,3-dioxolo[4,5-h]imidazo[1,2-c][2,3]benzodiazepin.

### Beispiel 52

### 5-(4-Fluorphenyl)-8-methyl-11H-1,3-dioxolo[4,5-h]imidazo[1,2-c][2,3]benzodiazepin

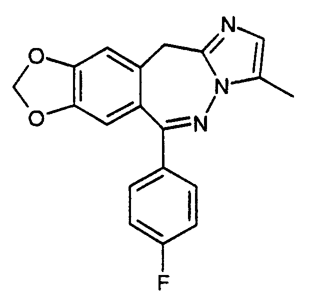

Zu einer Lösung von 6ml Fluorwasserstoff-Pyridin-Komplex (1:1) werden zunächst unter Argon 200mg 5-(4-Aminophenyl)-8-methyl-11H-1,3-dioxolo[4,5-h]imidazo[1,2-c][2,3]benzodiazepin und dann bei 0-5°C 51mg Natriumnitrit gegeben. Nach Rühren bei 5-10°C für 40min. werden 117mg Zinn(II)chlorid und 190mg Tetrabutylammoniumdihydrogentrifluorid zum Ansatz gegeben. Es wird dann 3h auf 100°C Badtemperatur erwärmt. Nach dem Abkühlen wird auf Eiswasser gegeben und dreimal mit Essigester und dreimal mit Methylenchlorid extrahiert. Die gesammelte organische Phase wird getrocknet, filtriert eingeengt und über Kieselgel zunächst mit Methylenchlorid:Ethanol = 10:1, dann ein zweites Mal mit Aceton:Essigester=3:1 und dann ein drittes Mal mit Methylenchlorid:Ethanol=95:5 chromatographiert. Man erhält 106mg 5-(4-Fluorphenyl)-8-methyl-11H-1,3-dioxolo[4,5-h]imidazo[1,2-c][2,3]benzodiazepin vom Schmelzpunkt 190°C.

### Beispiel 53

### 9-Brom-8-methyl-5-(4-nitrophenyl)-11H-1,3-dioxolo[4,5-h]imidazo[1,2-c][2,3]benzodiazepin

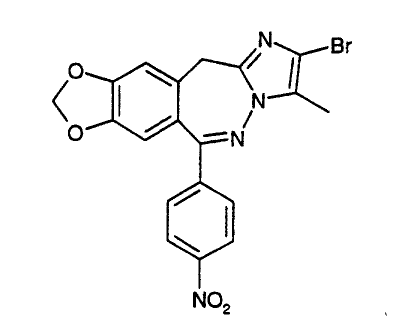

900mg 8-Methyl-5-(4-nitrophenyl)-11H-1,3-dioxolo[4,5-h]imidazo[1,2-c][2,3]benzodiazepin werden in 10ml Dimethylformamid mit 441mg N-Bromsuccinimid versetzt und für 1,5h bei Raumtemperatur gerührt. Nach Verdünnen mit 40ml Wasser wird das ausgefallene Produkt abgesaugt. man erhält 900mg 9-Brom-8-methyl-5-(4-nitrophenyl)-11H-1,3-dioxolo[4,5-h]imidazo[1,2-c][2,3]benzodiazepin.

In analoger Weise werden hergestellt:
8-Brom-9-methyl-5-(4-nitrophenyl)-11H-1,3-dioxolo[4,5-h]imidazo[1,2-c][2,3]benzodiazepin
2-Brom-8-methoxy-3-methyl-6-phenyl-11H-imidazo[1,2-c][2,3]benzodiazepin
2-Brom-8-methoxy-6-phenyl-3-(3-pyridyl)-11H-imidazo[1,2-c][2,3]benzodiazepin
8,9-Dibrom-5-(4-nitrophenyl)-11H-1,3-dioxolo[4,5-h]imidazo[1,2-c][2,3]benzodiazepin (unter Überschuss N-Bromsuccinimid)
3-Brom-8-methoxy-2-methyl-6-phenyl-11H-imidazo[1,2-c][2,3]benzodiazepin
8-Jod-5-(4-nitrophenyl)-11H-1,3-dioxolo[4,5-h]imidazo[1,2-c][2,3]benzodiazpein mit N-Jodsuccinimid

### Beispiel 54

### 2-Acetyl-3-(3-pyridyl)-8-methoxy-6-phenyl-11H-imidazo[1,2-c][2,3]benzodiazepin

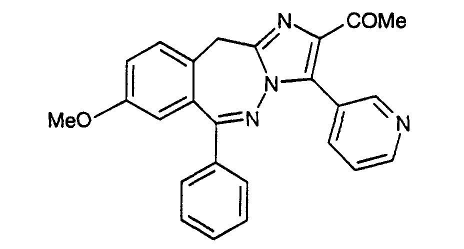

82mg 2-Brom-3-(3-pyridyl)-8-methoxy-6-phenyl-11H-imidazo[1,2-c][2,3]benzodiazepin werden in 3ml Toluol und 0,5ml Dimethylformamid mit 650mg (1-Ethoxyvinyl)tributylstannan und 10mg Palladium(0)tetrakistriphenylphosphin versetzt und 4h unter auf 120°C Badtemperatur erwärmt. Danach wird nochmals (1-Ethoxyvinyl)tributylstannan und 10mg Palladium(0)tetrakistriphenylphosphin zugegeben und 10h auf 120°C Badtemperatur erwärmt. Nach Abkühlen wird mit 2ml 1N-Salzsäure versetzt, 10min gerührt, mit Ammoniak alkalisch gestellt und mit Essigester ausgeschüttelt. Die Essigesterphase wird mit Wasser und gesättigter Kochsalzlösung gewaschen, getrocknet, filtriert und eingeengt. Nach Chromatographie des Rückstandes über Kieselgel mit Essigester als Elutionsmittel erhält man 20mg 2-Acetyl-3-(3-pyridyl)-8-methoxy-6-phenyl-11H-imidazo[1,2-c][2,3]benzodiazepin.

Analog wird erhalten: 2-Vinyl-3-(3-pyridyl)-8-methoxy-6-phenyl-11H-imidazo[1,2-c][2,3]benzodiazepin.
9-Propinyl-5-phenyl-11H-1,3-dioxolo[4,5-h]imidazo[1,2-c][2,3]benzodiazepin unter Zusatz von Cu(I)-Cokatalysator

### Beispiel 55

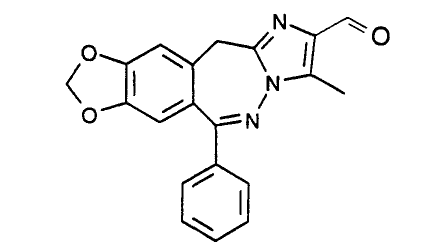

120 mg 9-Brom-8-methyl-5-phenyl-11H-1,3-dioxolo[4,5-h]imidazo[1,2-c][2,3]benzodiazepin werden in 15 ml Tetrahydrofuran bei - 78 °C mit 0,36 ml Butyllithium (Hexan 1 molar) versetzt und 15 Minuten gerührt. Es wird dann bei dieser Temperatur mit 0,6 ml Dimethylformamid versetzt und 15 Minuten gerührt. Nach Rühren auf Raumtemperatur wird mit Wasser versetzt, das Tetrahydrofuran abdestilliert und mit Ethylacetat extrahiert. Nach Abdestillieren des Lösemittels wird über Kieselgel mit Dichlormethan : Ethanol = 95 : 5 als Elutionsmittel chromatographiert. Man erhält 46 mg 9-Formyl-8-methyl-5-phenyl-11H-1,3-dioxolo[4,5-h]imidazo[1,2-c] [2,3]benzodiazpepin.

In grundsätzlich analoger Weise werden hergestellt:
9-(1-Hydroxyprop-1-yl)-8-methyl-5-phenyl-11H-1,3-dioxolo[4,5-h]imidazo[1,2-c][2,3]benzodiazepin
9-Ethyl-8-methyl-5-phenyl-11H-1,3-dioxolo[4,5-h]imidazo[1,2-c] [2,3]benzodiazepin
9-Methoxymethyl-8-methyl-5-phenyl-11H-1,3-dioxolo[4,5-h]imidazo[1,2-c][2,3]benzodiazepin

### Beispiel 56

100 mg 9-Jod-8-methyl-5-phenyl-11H-1,3-dioxolo[4,5-h]-imidazo[1,2-c][2,3]benzodiazepin werden in 4 ml Toluol und 1,5 ml Ethanol gelöst vorgelegt. Es werden 54 mg Diethyl-3-pyridyl-boran, 20 mg Tetrakis(triphenylphoshin)-palladium (0) und 0,8 ml einer 2-M Na₂CO₃-Lösung hinzugefügt und für 3 Stunden bei 110 °C gerührt. Nach Zugabe von Wasser wird gegen Essigsäureethylester extrahiert und die organische Phase eingeengt. Dieser Rückstand wird über Kieselgel mit Dichlormethan : Ethanol = 95 : 5 als Elutionsmittel chromatographiert. Man erhält 13 mg 8-Methyl-5-phenyl-9-(3-pyridyl)-11H-1,3-dioxolo[4,5-h]imidazo[1,2-c]benzodiazepin.

In analoger Weise wird aus
9-Jod-5-phenyl-11H-1,3-dioxolo[4,5-h]imidazo[1,2-c] [2,3]benzodiazepin hergestellt:
9-(3-Pyridyl)-5-phenyl-11H-1,3-dioxolo[4,5-h]imidazo[1,2-c][2,3]benzodiazepin

### Beispiel 57

100 mg 9-Jod-8-methyl-5-phenyl-11H-1,3-dioxolo[4,5-h]imidazo[1,2-c][2,3]benzodiazepin werden in 2 ml Dimethylformamid, 0,03 ml Triethylamin, 0,032 ml (30 mg) Diethylphosphit und 15 mg Tetrakis (triphenylphosphin) palladium (0) bei 100 °C für 2 Stunden gerührt. Anschließend wird mit 10 ml Wasser verdünnt und mit Essigsäureethylester extrahiert. Die eingeengte organische Phase wird über Kieselgel mit Dichlormethan : Ethanol = 95 : 5 als Elutionsmittel chromatographiert. Man erhält 10 mg 8-Methyl-5-phenyl-11H-1,3-dioxolo[4,5-h]imidazo[1,2-c] [2,3]benzodiazepin-9-phosphonsääurediethylester.

## Patentansprüche

1. Verbindungen der Formel I 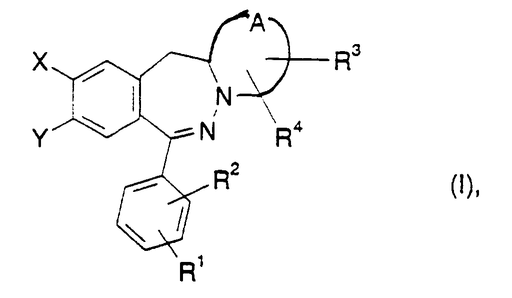 worin
R¹ und R² gleich oder verschieden sind und Wasserstoff, C₁ - C₆-Alkyl, Nitro, Halogen, Cyano, die Gruppe -NR⁸R⁹, -O-C₁₋₄-Alkyl, -CF₃, OH oder C₁₋₆-Alkanoyloxy,
R³ und R⁴ gleich oder verschieden sind und Wasserstoff, Halogen, C₁-C₆-Alkoxy, Hydroxy,Thiocyanato, C₁-C₆-Alkylthio, Cyano, COOR¹², PO₃R¹³R¹⁴, C₁₋₆-Alkanoyl, C₁₋₆-Alkanoyloxy, gegebenenfalls mit C₁₋₄-Alkoxy oder Phenyl substituiertes C₂₋₆-Alkynyl, gegebenenfalls mit C₁₋₄-Alkoxy oder Phenyl substituiertes C₂₋₆-Alkenyl, gegebenenfalls durch Halogen, Hydroxy, C₁-C₆-Alkoxy, C₁-C₆-Thioalkyl, NR¹⁰-R¹¹ substituiertes C₁ - C₆-Alkyl, C₃₋₇-Cycloalkyl oder einen gegebenenfalls mit Halogen, C₁₋₄-Alkyl und C₁₋₄-Alkoxy substituierten Aryl- oder Hetaryl-Rest,
R⁸ und R⁹ gleich oder verschieden sind und Wasserstoff, C₁ - C₆-Alkyl oder die Gruppe -CO-C₁₋₆-Alkyl,
R¹⁰ und R¹¹ gleich oder verschieden sind und Wasserstoff, C₁ - C₆-Alkyl oder C₁₋₆-Alkanoyl oder gemeinsam mit dem Stickstoffatom einen 5-7-gliedrigen gesättigten Heterocyclus bilden, der ein weiteres Sauerstoff-, Schwefel- oder Stickstoffatom enthalten und mit C₁₋₄-Alkyl und Phenyl substituiert sein kann,
R¹², R,¹³, R¹⁴ gleich oder verschieden sind und H oder C₁-C₆-Alkyl,
X Wasserstoff oder Halogen,
Y C₁₋₆-Alkoxy oder X und Y gemeinsam -O-(CH₂)ₙ-O-,
n 1, 2 oder 3 bedeuten und
A gemeinsam mit dem Stickstoff einen gesättigten oder ungesättigten fünfgliedrigen Heterocyclus bildet, der 1-3 Stickstoffatome und/oder ein Sauerstoffatom und/oder eine oder zwei Carbonylgruppen enthalten kann oder deren Isomere oder physiologisch verträglichen Salze.

2. 5-(4-Aminophenyl)-8-methyl-11H-1,3-dioxolo[4,5-h][1,2,4]triazolo[4,3-c](2,3]benzodiazepin
5-(4-Aminophenyl)-8-cyclopropyl-11H-1,3-dioxolo[4,5-h] [1,2,4]triazolo[4,3-c] [2,3]benzodiazepin
6-(4-Aminophenyl)-8-methoxy-3-propyl-11H-[1,2,4]triazolo[4,3-c][2,3]benzodiazepin
6-(4-Aminophenyl)-8-methoxy-3-ethyl-11H-[1,2,4]triazolo[4,3-c] [2,3]benzodiazepin
6-(4-Aminophenyl)-8-methoxy-3-cyclopropyl-11H-[1,2,4]triazolo[4,3-c] [2,3]benzodiazepin
5-(4-Aminophenyl)-9-methyl-11H-1,3-dioxolo[4,5-h]imidazo[1,2-c][2,3]benzodiazepin
5-(4-Aminophenyl)-8-cyclopropyl-11H-1,3-dioxolo[4,5-h]imidazo[1,2-c][2,3]benzodiazepin
5-(4-Aminophenyl)-8-methyl-11H-1,3-dioxolo[4,5-h]imidazo[1,2-c] [2,3]benzodiazepin
8-Cyclopropyl-5-(4-aminophenyl)-11H-1,3-dioxolo[4,5-h]imidazo[3,4-c][2,3]benzodiazepin
5-(4-Aminophenyl)-9-ethyl-11H-1,3-dioxolo[4,5-h]imidazo[1,2-c][2,3]benzodiazepin
5-(4-Aminophenyl)-8,9-dimethyl-11H-1,3-dioxolo[4,5-h]imidazo[1,2-c]-[2,3]benzodiazepin
8-Methoxy-3-methyl-6-phenyl-11H-imidazo[1,2-c][2,3]benzodiazepin
8-Methoxy-2-methyl-6-phenyl-11H-imidazo[1,2-c][2,3]benzodiazepin
8-Methoxy-3-methyl-6-phenyl-11H-imidazo[1,2-c][2,3]benzodiazepin
8-Methoxy-6-phenyl-3-(4-pyridyl)-11H-imidazo[1,2-c][2,3]benzodiazepin
8-Methoxy-6-phenyl-3-(2-pyridyl)-11H-imidazo[1,2-c][2,3]benzodiazepin
8-Methoxy-6-phenyl-3-(3-pyridyl)-11H-imidazo[1,2-c][2,3]benzodiazepin
2,3-Dimethyl-8-methoxy-6-phenyl-11H-imidazo[1,2-c][2,3]benzodiazepin
6-(4-Aminophenyl)-2,3-dimethyl-8-methoxy-11H-imidazo[1,2-c][2,3]benzodiazepin
6-(4-Aminophenyl)-8-methoxy-3-(2-pyridyl)-11H-imidazo[1,2-c][2,3]benzodiazepin
6-(4-Aminophenyl)-8-methoxy-3-(4-pyridyl)-11H-imidazo[1,2-c][2,3]benzodiazepin
5-(4-Aminophenyl)-8-(4-pyridyl)-11H-1,3-dioxolo[4,5-h]imidazo[1,2-c] [2,3]benzodiazepin
5-(4-Aminophenyl)-9-ethyl-8-methyl-11H-1,3-dioxolo[4,5-h]imidazo[1,2-c] [2,3]benzodiazepin
8-Methyl-5-phenyl-11H-1,3-dioxolo[4,5-h]imidazo[1,2-c][2,3]benzodiazepin
8,9-Dimethyl-5-phenyl-11H-1,3-dioxolo[4,5-h]imidazo[1,2-c] [2,3]benzodiazepin
8-Methoxy-2-methyl-6-phenyl-11H-imidazo[1,2-c] [2,3]benzodiazepin
8-Methoxy-6-phenyl-3-(4-pyridyl)-11H-imidazo[1,2-c][2,3]benzodiazepin gemäß Anspruch 1.

3. Verbindungen nach Anspruch 1,
worin A 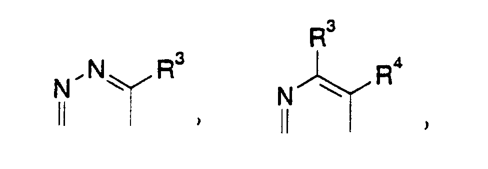 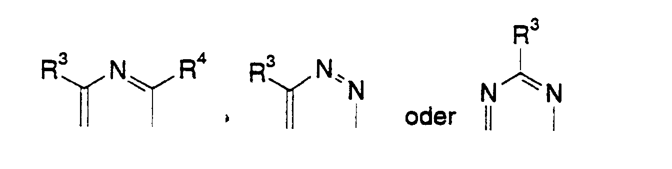 bedeutet.

4. Verbindungen nach Anspruch 1, worin A C₃-Alkylen, das mit R³ und R⁴ substituiert sein kann und bei dem 1,2 oder 3 Alkylengruppen durch Sauerstoff, Carbonyl oder -NR³- ersetzt sein können, bedeutet.

5. Verbindungen nach Anspruch 1, 3 und 4, worin R² Wasserstoff bedeutet.

6. Arzneimittel enthaltend eine Verbindung der Formel I gemäß Anspruch 1 oder deren physiologisch verträgliches Salz und pharmazeutisch übliche Träger- und Hilfsstoffe.

7. Verfahren zur Herstellung der Verbindung der Formel I gemäß Anspruch 1
**dadurch gekennzeichnet, daß** man
a) eine Verbindung der allgemeinen Formel II 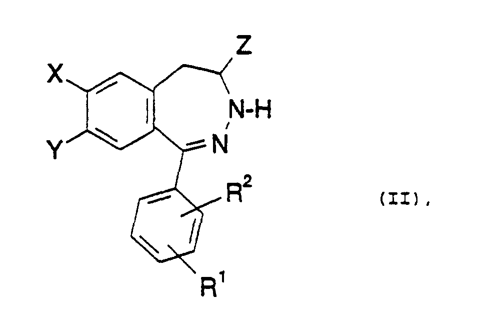 worin
R¹, R², X und Y die obige Bedeutung haben, cyclisiert durch Umsetzung von
α) Z COOC₁₋₆-Alkyl mit R³-N=C=O zu Verbindungen mit A in der Bedeutung -CO-NR³-CO-
β) Z = CH₂OH oder -CH₂-NHR³ mit Phosgen zu Verbindungen mit A in der Bedeutung - CH₂-O-CO- oder -CH₂-NR³-CO-
γ) Z = -CH₂OH mit R³-CO-R⁴ zu Verbindungen mit A in der Bedeutung -CH₂-O-CR³R⁴, worin R³ und R⁴ die obige Bedeutung haben,
b) eine Verbindung der Formel III oder IV 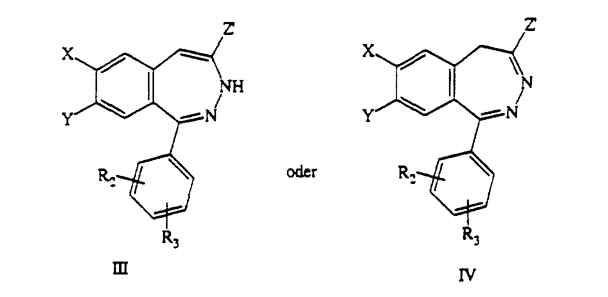 worin R¹, R², X und Y die obige Bedeutung haben, cyclisiert durch Umsetzung von
α) Z' = -CH=CH-COOC₁₋₆-Alkyl mit Borantrimethylamin-Komplex und Bortrifluoridetherat zu Verbindungen mit A in der Bedeutung -(CH₂)₃- und -(CH₂)₂-CO-
β) Z' = -CH=N-NH₂ in Gegenwart von Kupfersulfat zu Verbindungen mit A in der Bedeutung =CH-N=N-
γ) Z' = -S-C₁₋₄-Alkyl mit Hydrazinhydrat und Säureanhydriden oder mit Säurehydraziden zu Verbindungen mit A in der Bedeutung =N-N=CR³-
δ) Z' = -S-C₁₋₄-Alkyl mit α-Aminoacetalen zu Verbindungen mit A in der Bedeutung =N-CR³=CR⁴-
ε) Z' = CH₂OH in CH₂NH₂ überführt, dieses acyliert und zu Verbindungen mit A in der Bedeutung =CH-N=CR³- cyclisiert,
c) eine Verbindung der Formel V, 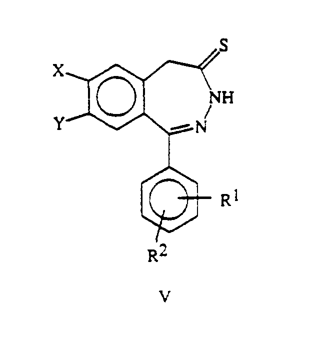
worin R¹, R², X und Y die oben gegebene Bedeutung haben, mit α-Aminoacetalen, α-Aminoketalen, H₂N-CH₂-C=C-R³ oder mit Ammoniak und α-Halogenketonen umsetzt, und anschließend gewünschtenfalls die Nitrogruppe R¹ und/oder R² reduziert, die Aminogruppe acyliert oder alkyliert oder in Halogen oder Hydroxy oder Cyano überführt oder desaminiert oder X gleichzeitig mit der Reduktion der Nitro-Gruppe oder nacheinander enthalogeniert oder Wasserstoff durch Halogen substituiert oder Halogen austauscht gegen ein anderes Halogen, -PO₃R¹³R¹⁴, Cyano, C₁₋₆-Alkanoyl, C₁₋₆-Alkanoyloxy, Hydroxy, gegebenenfalls substituiertes C₂₋₆-Alkynyl, gegebenenfalls substituiertes C₂₋₆-Alkenyl, gegebenenfalls substituiertes C₁₋₆-Alkyl, C₁₋₆-Alkoxyl CF₃, C₁₋₆-Thioalkyl, COOR¹² oder Y umethert oder die Isomeren trennt oder die Salze bildet.

8. Verbindungen der Formel IIa und IIIa, deren Isomere und Salze 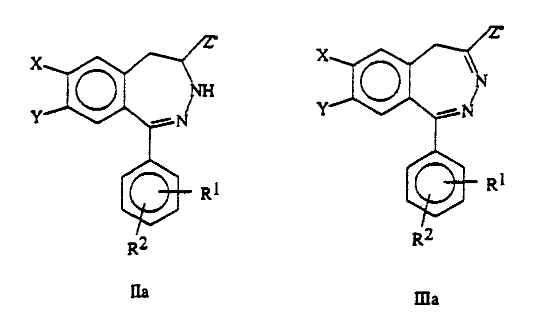 worin
R¹, R², X und Y die oben angegebene Bedeutung haben und
Z" -CH₂OH, -CHO, -COO-C₁₋₆-Alkyl, CH₂NHR³ oder 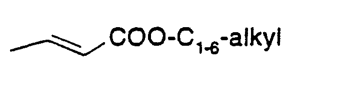 bedeutet und R³ die vorne genannte Bedeutung hat.

9. 5-(4-Nitrophenyl)-7H-1,3-dioxolo[4,5-h][2,3]benzodiazepin-8(9H)-on
5-(4-Nitrophenyl)-7H-1,3-dioxolo [4,5-h] [2,3]benzodiazepin-8 (9H)-thion
5-(4-Chlorphenyl)-7H-1,3-dioxolo[4,5-h][2,3]benzodiazepin-8(9H)-thion
5-(4-Fluorphenyl)-7H-1,3-dioxolo[4, 5-h] [2, 3]benzodiazepin-8(9H)-thion
5-(2-Fluorphenyl)-7H-1,3-dioxolo[4,5-h] [2,3]benzodiazepin-8(9H)-thion
5-(3-Chlorphenyl)-7H-1,3-dioxolo[4,5-h][2,3]benzodiazepin-8(9H)-thion
5-(2-Chlorphenyl)-7H-1,3-dioxolo[4,5-h][2,3]benzodiazepin-8(9H)-thion
5-Phenyl-7H-1,3-dioxolo[4,5-h][2,3]benzodiazepin-8(9H)-thion

10. Verwendung der Verbindungen nach Anspruch 8 und 9 zur Herstellung pharmakologisch wirksamer Verbindungen.

11. Verwendung der Verbindungen nach Anspruch 1 - 5 zur Herstellung eines Arzneimittels zur Behandlung von Krankheiten, die durch Hyperaktivität excitatorischer Aminosäuren ausgelöst werden.

12. Verwendung nach Anspruch 11 zur symptomatischen und präventiven Behandlung von Erkrankungen, die durch Überstimulation des AMPA-Rezeptors ausgelöst werden.

13. Verwendung nach Anspruch 11 - 12 zur Behandlung des Schlaganfalls, der Amyotrophen Lateralsklerose, der Olivopontocerebellaren Degeneration, des Zelluntergangs nach Hirntrauma und zur Prävention des postischämischen Zelluntergangs.

14. 8-(4-Nitrophenyl)-5-phenyl-11H-1,3-dioxolo[4,5-h][1,2,4]triazolo[4,3-c][2,3]benzodiazepin
8-(4-Aminophenyl)-5-phenyl-11H-1,3-dioxolo[4,5-h][1,2,4]triazolo[4,3-c][2,3]benzodiazepin

## Claims

1. Compounds of the formula I 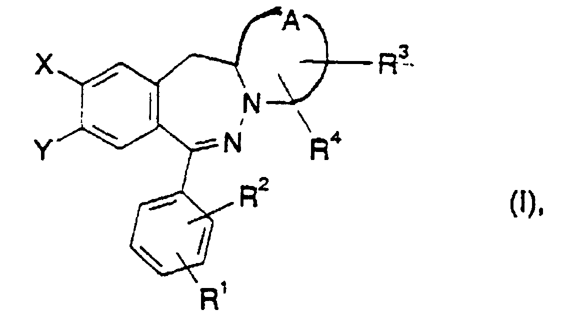 in which
R¹ and R² are identical or different and are hydrogen, C₁-C₆-alkyl, nitro, halogen, cyano, the group -NR⁸R⁹, -O-C₁₋₄-alkyl, -CF₃, OH or C₁₋₆-alkanoyloxy,
R³ and R⁴ are identical or different and are hydrogen, halogen, C₁-C₆-alkoxy, hydroxyl, thiocyanato, C₁-C₆-alkylthio, cyano, COOR¹², PO₃R¹³R¹⁴, C₁₋₆-alkanoyl, C₁₋₆-alkanoyloxy, C₂₋₆-alkynyl optionally substituted by C₁₋₄-alkoxy or phenyl, C₂₋₆-alkenyl optionally substituted by C₁₋₄-alkoxy or phenyl, C₁-C₆-alkyl optionally substituted by halogen, hydroxyl, C₁-C₆-alkoxy, C₁-C₆-thioalkyl or NR¹⁰R¹¹, C₃-C₇-cycloalkyl or an aryl or hetaryl radical optionally substituted by halogen, C₁₋₄-alkyl and C₁₋₄-alkoxy,
R⁸ and R⁹ are identical or different and are hydrogen, C₁-C₆-alkyl or the group -CO-C₁₋₆-alkyl,
R¹⁰ and R¹¹ are identical or different and are hydrogen, C₁-C₆-alkyl or C₁₋₆-alkanoyl or, together with the nitrogen atom, form a 5- to 7-membered saturated heterocycle which can contain a further oxygen, sulphur or nitrogen atom and can be substituted by C₁₋₄-alkyl and phenyl,
R¹² ,R¹³ ,R¹⁴ are identical or different and are H or C₁-C₆-alkyl,
X is hydrogen or halogen,
Y is C₁₋₆-alkoxy or X and Y together are -O-(CH₂)ₙ-O-,
n is 1, 2 or 3 and
A, together with the nitrogen, forms a saturated or unsaturated five-membered heterocycle which can contain 1-3 nitrogen atoms and/or an oxygen atom and/or one or two carbonyl groups, or their isomers or physiologically tolerable salts.

2. 5-(4-Aminophenyl)-8-methyl-11H-1,3-dioxolo-[4,5-h][1,2,4]triazolo[4,3-c] [2,3]benzodiazepine
5-(4-aminophenyl)-8-cyclopropyl-11H-1,3-dioxolo[4,5-h]-[1,2,4]triazolo[4,3-c][2,3]benzodiazepine
6-(4-aminophenyl)-8-methoxy-3-propyl-11H-[1,2,4]triazolo[4,3-c] [2,3]benzodiazepine
6-(4-aminophenyl)-8-methoxy-3-ethyl-11H-[1,2,4]triazolo[4,3-c][2,3]benzodiazepine
6-(4-aminophenyl)-8-methoxy-3-cyclopropyl-11H-[1,2,4]triazolo[4,3-c][2,3]benzodiazepine
5-(4-aminophenyl)-9-methyl-11H-1,3-dioxolo[4,5-h]-imidazo[1,2-c][2,3]benzodiazepine
5-(4-aminophenyl)-8-cyclopropyl-11H-1,3-dioxolo[4,5-h]-imidazo[1,2-c][2,3]benzodiazepine
5-(4-aminophenyl)-8-methyl-11H-1,3-dioxolo[4,5-h]-imidazo[1,2-c][2,3]benzodiazepine
8-cyclopropyl-5-(4-aminophenyl)-11H-1,3-dioxolo[4,5-h]-imidazo[3,4-c][2,3]benzodiazepine
5-(4-aminophenyl)-9-ethyl-11H-1,3-dioxolo[4,5-h]-imidazo[1,2-c][2,3]benzodiazepine
5-(4-aminophenyl)-8,9-dimethyl-11H-1,3-dioxolo[4,5-h]-imidazo[1,2-c][2,3]benzodiazepine
8-methoxy-3-methyl-6-phenyl-11H-imidazo[1,2-c][2,3]-benzodiazepine
8-methoxy-2-methyl-6-phenyl-11H-imidazo[1,2-c] [2,3]-benzodiazepine
8-methoxy-3-methyl-6-phenyl-11H-imidazo[1,2-c][2,3]-benzodiazepine
8-methoxy-6-phenyl-3-(4-pyridyl)-11H-imidazo[1,2-c]-[2,3]benzodiazepine
8-methoxy-6-phenyl-3-(2-pyridyl)-11H-imidazo[1,2-c]-[2,3]benzodiazepine
8-methoxy-6-phenyl-3-(3-pyridyl)-11H-imidazo[1,2-c]-[2,3]benzodiazepine
2,3-dimethyl-8-methoxy-6-phenyl-11H-imidazo[1,2-c]-[2,3]benzodiazepine
6-(4-aminophenyl)-2,3-dimethyl-8-methoxy-11H-imidazo[1,2-c][2,3]benzodiazepine
6-(4-aminophenyl)-8-methoxy-3-(2-pyridyl)-11H-imidazo[1,2-c][2,3]benzodiazepine
6-(4-aminophenyl)-8-methoxy-3-(4-pyridyl)-11H-imidazo[1,2-c][2,3]benzodiazepine
5- (4-aminophenyl)-8-(4-pyridyl)-11H-1,3-dioxolo[4,5-h]-imidazo[1,2-c][2,3]benzodiazepine
5-(4-aminophenyl)-9-ethyl-8-methyl-11H-1,3-dioxolo-[4,5-h]imidazo[1,2-c][2,3]benzodiazepine
8-methyl-5-phenyl-11H-1,3-dioxolo[4,5-h]imidazo[1,2-c]-[2,3]benzodiazepine
8,9-dimethyl-5-phenyl-11H-1,3-dioxolo[4,5-h]imidazo-[1,2-c][2,3]benzodiazepine
8-methoxy-2-methyl-6-phenyl-11H-imidazo[1,2-c][2,3]-benzodiazepine
8-methoxy-6-phenyl-3-(4-pyridyl)-11H-imidazo[1,2-c]-[2,3]benzodiazepine
according to Claim 1.

3. Compounds according to Claim 1,
in which A is

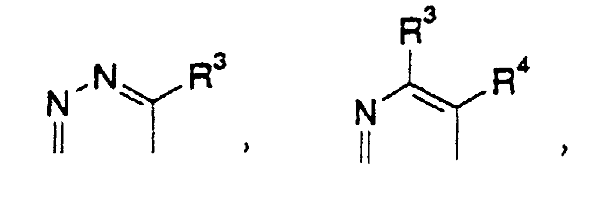

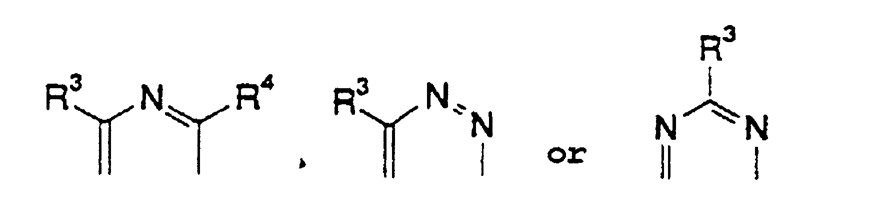

4. Compounds according to Claim 1, in which A is C₃-alkylene which can be substituted by R³ and R⁴ and in which 1, 2 or 3 alkylene groups can be replaced by oxygen, carbonyl or -NR³-.

5. Compounds according to Claim 1, 3 and 4, in which R² is hydrogen.

6. Pharmaceutical comprising a compound of the formula I according to Claim 1 or its physiologically tolerable salt and pharmaceutically customary vehicles and excipients.

7. Process for the preparation of the compound of the formula I according to Claim 1, **characterized in that**
a) a compound of the general formula II 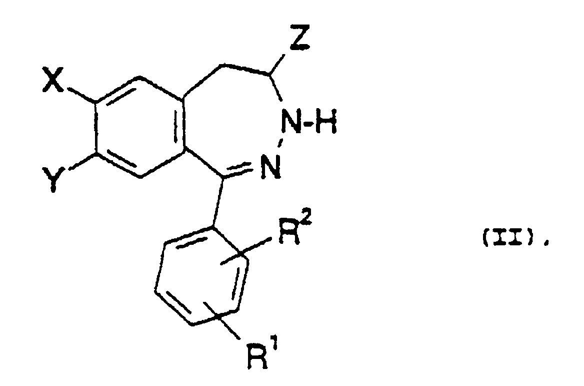 in which
R¹, R², X and Y have the above meaning, is cyclized by reaction of
α) Z = COOC₁₋₆-alkyl with R³-N=C=O to give compounds where A has the meaning -CO-NR³-CO-
β) Z = CH₂OH or -CH₂-NHR³ with phosgene to give compounds where A has the meaning -CH₂-O-CO- or -CH₂-NR³-CO-
γ) Z = -CH₂OH with R³-CO-R⁴ to give compounds where A has the meaning -CH₂-O-CR³R⁴, in which R³ and R⁴ have the above meaning, 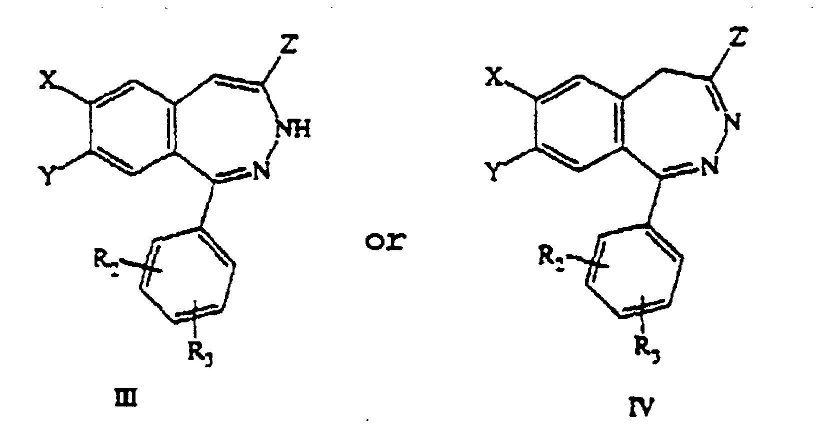
b) a compound of the formula III or IV
in which R¹, R², X and Y have the above meaning, is cyclized by reaction of
α) Z' = -CH=CH-COOC₁₋₆-alkyl with borane-trimethylamine complex and boron trifluoride etherate to give compounds where A has the meaning -(CH₂)₃- and -(CH₂)₂-CO-
β) Z' = -CH=N-NH₂ in the presence of copper sulphate to give compounds where A has the meaning =CH-N=N-
γ) Z' = -S-C₁₋₄-alkyl with hydrazine hydrate and acid anhydrides or with acid hydrazides to give compounds where A has the meaning =N-N=CR³-
δ) Z' = -S-C₁₋₄-alkyl with α-aminoacetals to give compounds where A has the meaning =N-CR³=CR⁴-
ε) Z' = CH₂OH is converted into CH₂NH₂, this is acylated and cyclized to give compounds where A has the meaning =CH-N=CR³-,
c) a compound of the formula V, 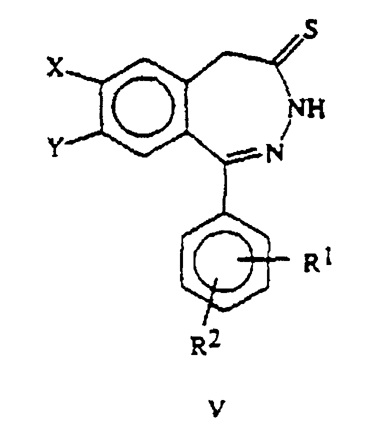 in which R¹, R², X and Y have the meaning given above, is reacted with α-aminoacetals, α-aminoketals, H₂N-CH₂=C-R³ or with ammonia and α-haloketones, and subsequently if desired the nitro group R¹ and/or R² is/are reduced, the amino group is acylated or alkylated or is converted into halogen or hydroxyl or cyano or is deaminated or dehalogenated simultaneously to the reduction of the nitro group or successively or hydrogen is substituted by halogen or halogen is replaced by another halogen, -PO₃R¹³R¹⁴, cyano, C₁₋₆-alkanoyl, C₁₋₆-alkanoyloxy, hydroxyl, optionally substituted C₂₋₆-alkynyl, optionally substituted C₂₋₆-alkenyl, optionally substituted C₁₋₆-alkyi, C₁₋₆-alkoxy, CF₃, C₁₋₆-thioalkyl, COOR¹² or Y is transetherified or the isomers are separated or the salts are formed.

8. Compounds of the formula IIa and IIIa, their isomers and salts 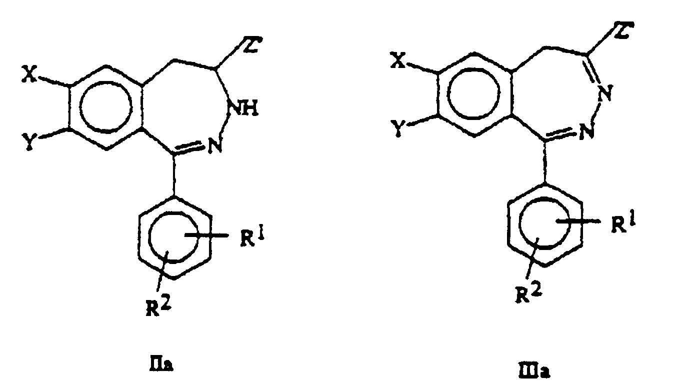 in which
R¹, R², X and Y have the meaning indicated above and Z" is -CH₂OH, -CHO, -COO-C₁₋₆-alkyl, CH₂NHR³ or 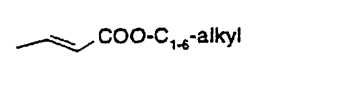 and R³ has the meaning mentioned at the beginning.

9. 5-(4-Nitrophenyl)-7H-1,3-dioxclo[4,5-h][2,3]-benzodiazepine-8(9H)-one
5-(4-nitrophenyl)-7H-1,3-dioxolo[4,5-h][2,3]-benzodiazepine-8(9H)-thione
5-(4-chlorophenyl)-7H-1,3-dioxolo[4,5-h][2,3]-benzodiazepine-8(9H)-thione
5-(4-fluorophenyl)-7H-1,3-dioxolo[4,5-h][2,3]-benzodiazepine-8(9H)-thione
5-(2-fluorophenyl)-7H-1,3-dioxolo[4,5-h][2,3]-benzodiazepine-8(9H)-thione
5-(3-chlorophenyl)-7H-1,3-dioxolo[4,5-h][2,3]-benzodiazepine-8(9H)-thione
5-(2-chlorophenyl)-7H-1,3-dioxolo[4,5-h] [2,3]-benzodiazepine-8(9H)-thione
5-phenyl-7H-1,3-dioxolo[4,5-h][2,3]benzodiazepine-8(9H)-thione

10. Use of the compounds according to Claim 8 and 9 for the production of pharmacologically active compounds.

11. Use of the compounds according to Claim 1 - 5 for the production of a medicament for the treatment of diseases which are caused by hyperactivity of excitatory amino acids.

12. Use according to Claim 11 for the symptomatic and preventive treatment of disorders which are caused by overstimulation of the AMPA receptor.

13. Use according to Claim 11 - 12 for the treatment of stroke, of amyotrophic lateral sclerosis, of olivopontocerebellar degeneration, of cell death after cerebral trauma and for the prevention of postischaemic cell death.

14. 8-(4-Nitrophenyl)-5-phenyl-11H-1,3-dioxolo[4,5-h][1,2,4]triazolo[4,3-c][2,3]benzodiazepine
8-(4-aminophenyl)-5-phenyl-11H-1,3-dioxolo[4,5-h][1,2,4]triazolo[4,3-c][2,3]benzodiazepine

## Revendications

1. Composés de formule 1 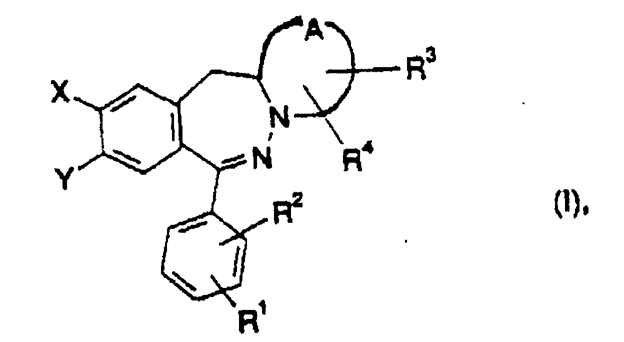 dans laquelle
R¹ et R² sont identiques ou différents et signifient un hydrogène, un alkyle en C₁ à C₆, un nitro, un halogène, un cyano, le groupement -NR⁸R⁹, -O-(alkyle en C₁₋₄) , -CF₃, OH ou un alcanoyloxy en C₁₋₆,
R³ et R⁴ sont identiques ou différents et signifient un hydrogène, un halogène, un alkoxy en C₁ à C₆, un hydroxy, un thiocyanato, un alkylthio en C₁ à C₆, un cyano, COOR¹², PO₃R¹³R¹⁴, un alcanoyle en C₁₋₆, un alcanoyloxy en C₁₋₆, un alcynyle en C₂₋₆ le cas échéant substitué par un alkoxy en C₁₋₄ ou par un phényle, un alcényle en C₂₋₆ le cas échéant substitué par un alkoxy en C₁₋₄ ou par un phényle, un alkyle en C₁ à C₆ le cas échéant substitué par un halogène, par un hydroxy, par un alkoxy en C₁ à C₆, par un thioalkyle en C₁ à C₆, par NR¹⁰-R¹¹, un cycloalkyle en C₃₋₇ ou un radical aryle ou hétéroaryle le cas échéant substitué par un halogène, par un alkyle en C₁₋₄ et par un alkoxy en C₁₋₄,
R⁸ et R⁹ sont identiques ou différents et signifient un hydrogène, un alkyle en C₁ à C₆ ou le groupement -CO-(alkyle en C₁₋₆),
_{R}¹⁰ et R¹¹ sont identiques ou différents et signifient un hydrogène, un alkyle en C₁ à C₆ ou un alcanoyle en C₁₋₆ ou forment ensemble avec l'atome d'azote un hétérocycle saturé de 5 à 7 membres, qui peut contenir un autre atome d'oxygène, de soufre ou d'azote et qui peut être substitué par un alkyle en C₁₋₄ et un phényle,
R¹², R¹³, R¹⁴ sont identiques ou différents et signifient H ou un alkyle en C₁ à C₆,
X signifie un hydrogène ou un halogène,
Y signifie un alkoxy en C₁₋₆ ou X et Y signifient ensemble -O-(CH₂)ₙ-O-,
n signifie 1, 2 ou 3 et
A forme ensemble avec l'azote un hétérocycle saturé ou insaturé à cinq membres, qui peut contenir 1 à 3 atomes d'azote et/ou un atome d'oxygène et/ou un ou deux groupements carbonyle, ou leurs isomères ou leurs sels physiologiquement compatibles.

2. 5-(4-aminophényl)-8-méthyl-11H-1,3-dioxolo[4,5-h][1,2,4]triazolo[4,3-c][2,3]benzodiazépine
5-(4-aminophényl)-8-cyclopropyl-11H-1,3-dioxolo[4,5-h][1,2,4]triazolo[4,3-c][2,3]benzodiazépine,
6-(4-aminophényl)-8-méthoxy-3-propyl-11H-[1,2,4]triazolo[4,3-c] [2,3]benzodiazépine
6-(4-aminophényl)-8-méthoxy-3-éthyl-11H-[1,2,4]triazolo[4,3-c][2,3]benzodiazépine
6-(4-aminophényl)-8-méthoxy-3-cyclopropyl-11H-[1,2,4]triazolo[4,3-c][2,3]benzodiazépine
5-(4-aminophényl)-9-méthyl-11H-1,3-dioxolo[4,5-h]imidazo[1,2-c][2,3]benzodiazépine
5-(4-aminophényl)-8-cyclopropyl-11H-1,3-dioxolo[4,5-h]imidazo[1,2-c][2,3]benzodiazépine
5-(4-aminophényl)-8-mnéthyl-11H-1,3-dioxolo[4,5-h]imidazo[1,2-c][2,3]benzodiazépine
8-cyclopropyl-5-(4-aminophényl)-11H-1,3-dioxolo[4,5-h]imidazo[3,4-c][2,3]benzodiazépine
5-(4-aminophényl)-9-éthyl-11H-1,3-dioxolo[4,5-h]imidazo[1,2-c][2,3]benzodiazépine
5-(4-aminophényl)-8,9-diméthyl-11H-1,3-dioxolo[4,5-h]imidazo[1,2-c][2,3]benzodiazépine
8-méthoxy-3-méthyl-6-phényl-11H-imidazo[1,2-c][2,3]benzodiazépine
8-méthoxy-2-méthyl-6-phényl-11H-imidazo[1,2-c][2,3]benzodiazépine
8-méthoxy-3-méthyl-6-phényl-11H-imidazo[1,2-c][2,3]benzodiazépine
8-méthoxy-6-phényl-3-(4-pyridyl)-11H-imidazo[1,2-c][2,3]benzodiazépine
8-méthoxy-6-phényl-3-(2-pyridyl)-11H-imidazo[1,2-c][2,3]benzodiazépine
8-méthoxy-6-phényl-3-(3-pyridyl)-11H-imidazo[1,2-c][2,3]benzodiazépine
2,3-diméthyl-8-méthoxy-6-phényl-11H-imidazo[1,2-c][2,3]benzodiazépine
6-(4-aminophényl)-2,3-diméthyl-8-méthoxy-11H-imidazo[1,2-c][2,3]benzodiazépine
6-(4-aminophényl)-8-méthoxy-3-(2-pyridyl)-11H-imidazo[1,2-c][2,3]benzodiazépine
6-(4-aminophényl)-8-méthoxy-3-(4-pyridyl)-11H-imidazo[1,2-c] [2,3]benzodiazépine
5-(4-aminophényl)-8-(4-pyridyl)-11H-1,3-dioxolo[4,5-h]imidazo[1,2-c][2,3]benzodiazépine
5-(4-aminophényl)-9-éthyl-8-méthyl-11H-1,3-dioxolo[4,5-h]imidazo[1,2-c][2,3]benzodiazépine
8-méthyl-5-phényl-11H-1,3-dioxolo[4,5-h]imidazo[1,2-c][2,3]benzodiazépine
8,9-diméthyl-5-phényl-11H-1,3-dioxolo[4,5-h]imidazo[1,2-c][2,3]benzodiazépine
8-méthoxy-2-méthyl-6-phényl-11H-imidazo[1,2-c][2,3]benzodiazépine
8-méthoxy-6-phényl-3-(4-pyridyl)-11H-imidazo[1,2-c][2,3]benzodiazépine selon la revendication 1.

3. Composés selon la revendication 1,
dans lesquels A signifie

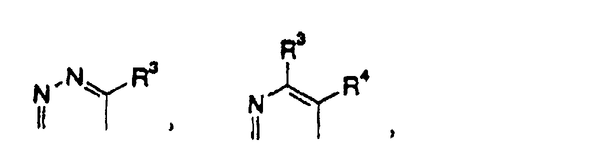

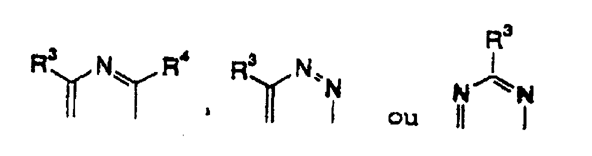

4. Composés selon la revendication 1, dans lesquels A signifie un alkylène en C₃, qui peut être substitué par R³ et R⁴ et dans lequel 1, 2 ou 3 groupements alkylène peuvent être remplacés par de l'oxygène, par un carbonyle ou par -NR³-.

5. Composés selon les revendications 1, 3 et 4, dans lesquels R² signifie un hydrogène.

6. Médicament contenant un composé de formule I selon la revendication 1 ou son sel physiologiquement compatible et des substances support et adjuvants pharmaceutiquement usuels.

7. Procédé pour la préparation du composé de formule I selon la revendication 1, caractérisé
a) en ce qu'on cyclise un composé de formule générale II 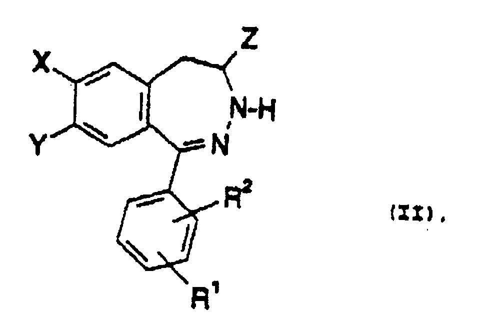 dans laquelle
R¹, R², X et Y ont la signification susmentionnée, par transformation de
α) Z = COO(alkyle en C₁₋₆) avec R³-N=C=O en composés avec A ayant la signification -CO-NR³-CO-,
β) Z = CH₂OH ou -CH₂-NHR³ avec du phosgène en composés avec A ayant la signification -CH₂-O-CO- ou -CH₂-NR³-CO-
γ) Z = -CH₂OH avec R³-CO-R⁴ en composés avec A ayant la signification -CH₂-O-CR³R⁴, où R³ et R⁴ ont la signification susmentionnée,
b) en ce qu'on cyclise un composé de formule III ou IV 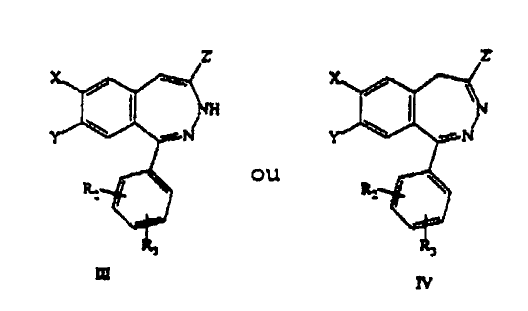 dans lesquelles R¹, R², X et Y ont la signification susmentionnée, par transformation de
α) Z' = -CH=CH-COO(alkyle en C₁₋₆) avec un complexe de borantriméthylamine et de l'éthérate de borotrifluorure en composés avec A ayant la signification -(CH₂)₃- et -(CH₂)₂-CO-
β) Z' = -CH=N-NH₂ en présence de sulfate de cuivre en composés avec A ayant la signification =CH-N=N-,
γ) Z' = -S-(alkyle en C₁₋₄) avec de l'hydrate d'hydrazine et des anhydrides d'acide ou des hydrazides d'acide en composés avec A ayant la signification =N-N=CR³-
δ) Z' = -S-(alkyle en C₁₋₄) avec des α-aminoacétals en composés avec A ayant la signification =N-CR³=CR⁴-
ε) en transformant Z' = CH₂OH en CH₂NH₂, en acylant celui-ci et en le cyclisant en composés avec A ayant la signification =CH-N=CR³-,
c) en ce qu'on transforme un composé de formule V 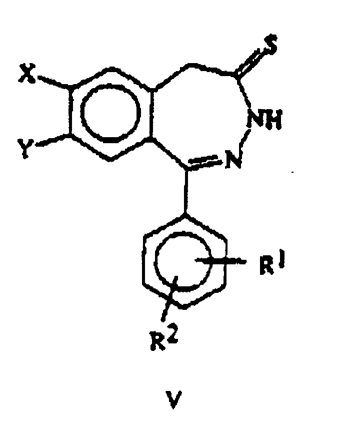
dans laquelle R¹, R², X et Y ont la signification susmentionnée, avec des α-aminoacétals, des α-aminocétals, H₂N-CH₂-C≡C-R³ ou avec de l'ammoniac et des α-halogénocétones et on réduit ensuite si souhaité le groupement nitro R¹ et/ou R², on acyle ou alkyle ou on transforme en halogène ou hydroxy ou cyano
ou on désamine le groupement amino ou on déshalogène X simultanément avec la réduction du groupement nitro ou successivement, ou on substitue un hydrogène par un halogène ou on remplace un halogène par un autre halogène, -PO₃R¹³R¹⁴, un cyano, un alcanoyle en C₁₋₆, un alcanoyloxy en C₁₋₆, un hydroxy, un alcynyle en C₂₋₆ le cas échéant substitué, un alcényle en C₂₋₆ le cas échéant substitué, un alkyle en C₁₋₆ le cas échéant substitué, un alkoxy en C₁₋₆, CF₃, un thioalkyle en C₁₋₆, COOR¹² ou on transéthérifie Y ou on sépare les isomères ou on forme les sels.

8. Composés de formules IIa et IIIa, leurs isomères et sels 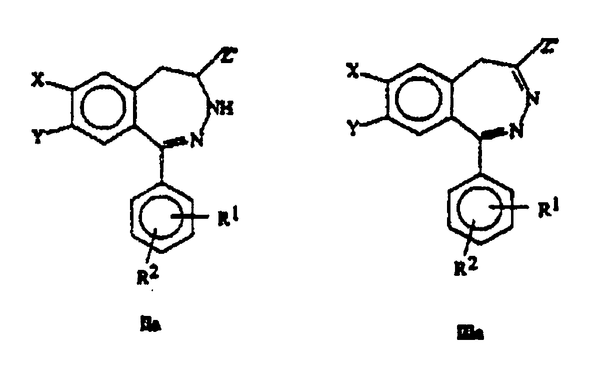 dans lesquelles
R¹, R², X et Y ont la signification susmentionnée et
Z" signifie -CH₂OH, -CHO, -COO-(alkyle en C₁-₆), CH₂NHR³ ou 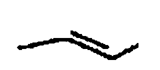 COO-(alkyle en C₁₋₆) et R³ a la signification susmentionnée.

9. 5-(4-nitrophényl)-7H-1,3-dioxolo[4,5-h][2,3]benzodiazépine-8(9H)-one
5-(4-nitrophényl)-7H-1,3-dioxolo[4,5-h][2,3]benzodiazépine-8(9H)-thione
5-(4-chlorophényl)-7H-1,3-dioxolo[4,5-h][2,3]benzodiazépine-8(9H)-thione
5-(4-fluorophényl)-7H-1,3-dioxolo[4,5-h][2,3]benzodiazépine-8(9H)-thione
5-(2-fluorophényl) -7H-1,3-dioxolo[4,5-h][2,3]benzodiazépine-8(9H)-thione
5-(3-chlorophényl)-7H-1,3-dioxolo[4,5-h][2,3]benzodiazépine-8(9H)-thione
5-(2-chlorophényl)-7H-1,3-dioxolo[4,5-h][2,3]benzodiazépine-8(9H)-thione
5-phényl-7H-1,3-dioxolo[4,5-h]-[2,3]benzodiazépine-8(9H)-thione

10. Utilisation des composés selon les revendications 8 et 9 pour la préparation de composés pharmacologiquement actifs.

11. Utilisation des composés selon les revendications 1 à 5 pour la préparation d'un médicament pour le traitement de maladies qui sont déclenchées par une hyperactivité des acides aminés excitateurs.

12. Utilisation selon la revendication 11 pour le traitement symptomatique et préventif de maladies qui sont déclenchées par une hyperstimulation du récepteur AMPA.

13. Utilisation selon les revendications 11 à 12 pour le traitement de l'attaque d'apoplexie, la sclérose latérale amyotrophique, la dégénérescence olivo-ponto-cérébelleuse, la mort cellulaire après un traumatisme cérébral et pour la prévention de la mort cellulaire post-ischémique.

14. 8-(4-Nitrophényl)-5-phényl-11H-1,3-dioxolo[4,5-h][1,2,4]triazolo[4,3-c][2,3]benzodiazépine
8-(4-aminophényl)-5-phényl-11H-1,3-dioxolo[4,5-h][1,2,4]triazolo[4,3-c][2,3]benzodiazépine
